# EUROPÄISCHE PATENTSCHRIFT

(11) **EP 0 792 881 B1**
(45) Veröffentlichungstag und Bekanntmachung des Hinweises auf die Patenterteilung: **20.06.2001**
(21) Anmeldenummer: 97250039.1
(22) Anmeldetag: 21.02.1997
(51) Int. Cl.: C07D 493/08, A61K 6/083, C07D 493/18

(54) **Funktionalisierte bicyclische (Meth)acrylate**
Functionalised bicyclic (meth)acrylates
(Meth)acrylates bicycliques functionalisés

(30) Priorität: 22.02.1996 DE 19608316
(43) Veröffentlichungstag der Anmeldung: 03.09.1997
(73) Patentinhaber: Ivoclar Vivadent AG, 9494 Schaan (LI)
(72) Erfinder: Moszner, Norbert, Prof. Dr., 9492 Eschen (LI); Rheinberger, Volker, Dr., 9490 Vaduz (LI); Vogel, Karin, Dipl.-Ing., 9493 Mauren (LI); Zeuner, Frank, Dr., 9490 Vaduz (LI)
(74) Vertreter: UEXKÜLL & STOLBERG

(56) Entgegenhaltungen:
- US-A- 3 681 438
- US-A- 4 054 233
- US-A- 4 808 638

## Beschreibung

Die Erfindung betrifft funktionalisierte bicyclische (Meth)acrylate, ein Verfahren zu deren Herstellung, deren Verwendung insbesondere als Dentalmaterial, ein diese enthaltendes Dentalmaterial sowie aus diesen erhältliche Polymere und Copolymere.

Bicyclische Verbindungen mit Bicyclo[2.2.1]hept-2-enyl- (Norbornenyl) oder 7-Oxa-bicyclo[2.2.1]hept-2-enyl-Gruppen sowie solche mit Bicyclo[2.2.1]hept-2,5-dienyl-Gruppen (Norbornadienyl) sind bekannt und finden als Monomere für die ringöffnende Metathesepolymerisation besonderes Interesse (D.J. Brunelle (Ed.), Ring-Opening Polymerization, Hanser Pub. Munich etc. 1993, S. 129). Darüber hinaus werden Norbornen-Verbindungen in der US-A-4 808 638 auch als reaktive En-Komponenten für die schrumpfungsarme Thiol-En-Polymerisation beschrieben.

Monomere mit mindestens zwei unterschiedlichen polymerisierbaren Gruppen werden als ambifunktionelle Monomere oder Hybridmonomere bezeichnet, und sie ermöglichen die gezielte Synthese von vernetzbaren Polymeren. In Abhängigkeit von der Art der polymerisierbaren Gruppen kann die ein- oder zweistufige Synthese von Polymernetzwerken durch Kombination beispielsweise von radikalischer und kationischer Vinylpolymerisation, z.B. bei Vinyloxyethylmethacylat, oder von anionischer Gruppentransferpolymerisation und radikalischer Polymerisation, z.B. bei Acryloyloxyethylmethacrylat, erreicht werden. In diesem Zusammenhang ist auch die ringöffnende Metathesepolymerisation von Cyclooct-5-enylmethacrylat bekannt, die zu radikalisch vernetzbaren Polymeren führt (B.R. Maughon, R.H. Grubbs, Amer. Chem. Soc. Polym. Div., Polym. Prep. 36 (1) (1995) 471).

Bicyclische Methacrylate sind ebenfalls bekannt und sie haben verschiedenartige Verwendung gefunden. So wird z.B. in der US-A-4 054 233 die Synthese und Polymerisation von Norbornenylmethylmethacrylat im Zusammenhang mit peroxidisch vernetzbaren Schichten beschrieben. Norbornenylmethylmethacrylat oder Borneolmethacrylat finden auch bei der Herstellung von PVC mit verbesserter Wärmestabilität Anwendung (vgl. SU-A-1 776 673, Chem. Abstract, 119, 272563). Adhäsive Polymere auf der Basis von Umsetzungsprodukten von 5-Norbornen-2,3-dicarbonsäureanhydrid mit Hydroxyalkyl-(meth)acrylaten, z.B. 2-Hydroxyethyl(methacrylat), werden in der CA-A-1 013 095 erwähnt. Ferner ist es auch bekannt, daß radikalisch vernetzbare Polyimide über 7-Oxa-5,6-dicarboxyimid-N-yl-bicyclo[2.2.1]-hept-2-enacrylat zugänglich sind (T.M. Pyriadi, I.U,. Altmamimi, Macromol. Rep. A31 (1994) 191). Schließlich sind auch Additionsprodukte der Umsetzung von Dicyclopentadien mit (Meth)acrylsäure bekannt (S. Teshigahara, Y. Kano, Toso Kenkyu Hokoko, 35 (1991) 47, Chem. Abstr. 116 84740).

Der Erfindung liegt die Aufgabe zugrunde, funktionalisierte bicyclische (Meth)acrylate bereitzustellen, die einfach herstellbar und bei Raumtemperatur radikalische härtbar sind, durch ringöffnende Metathesepolymerisation (RÖMP) polymerisiert werden können und sich insbesondere als Dentalmaterial oder Bestandteil davon und vor allem als haftungserhöhende Komponente von Dentinadhäsiven eignen.

Diese Aufgabe wird durch die funktionalisierten bicyclischen (Meth)acrylate nach den Ansprüchen 1 bis 8 gelöst.

Gegenstand der vorliegenden Erfindung sind ebenfalls das Verfahren zur Herstellung der funktionalisierten bicyclischen (Meth)acrylate nach Anspruch 9, deren Verwendung nach den Ansprüchen 10 und 11, Dentalmaterialien mit Gehalt an diesen nach den Ansprüchen 12 und 13 sowie Polymere oder Copolymere nach Anspruch 14, welche durch Polymerisation oder Copolymerisation der funktionalisierten bicyclischen (Meth(Meth)acrylate erhältlich sind.

Bei den erfindungsgemäßen funktionalisierten bicyclischen (Meth)acrylaten handelt es sich um Verbindungen der folgenden Formeln I oder II, dazu stereoisomere Verbindungen und beliebige Mischungen von allen diesen, 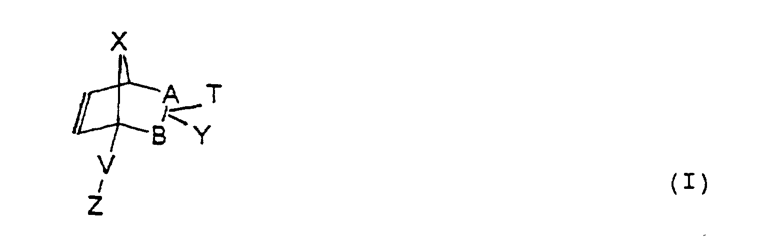 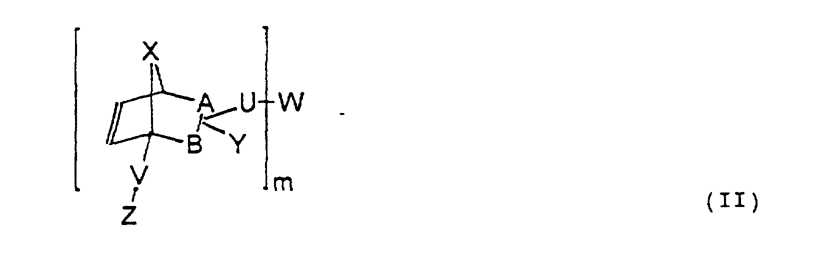 wobei A-B, T, U, V, W, X, Y, Z, R, R¹, R², R³, R⁴, n und m unabhängig voneinander die folgenden Bedeutungen haben:
- A-B =: C-C oder C=C;
- X =: CH₂, O, N-CO-OR, N-COR, N-CONR₂ oder N-SO₂R,
wobei
die einzelnen Reste R unabhängig voneinander = substituiertes oder unsubstituiertes C₁- bis C₁₂-Alkyl oder C₆-bis C₁₄-Aryl;
- Z =: CH₂=CH-CO- oder CH₂=C(CH₃)-CO-;
- V =: C₁- bis C₆-Alkylenoxy, CH₂-S, CH₂-NH oder COO-(C₁- bis C₆)-Alkylenoxy;
- Y =: H, C₁- bis C₁₂-Alkyl, C6- bis C₁₄-Aryl, Halogen, NO₂, NR¹₂, OR¹, CN, CO-R¹, CO-NR¹₂, CO-OR¹, SR¹, SO₂R¹ oder SO₃R¹,
wobei
die einzelnen Reste R¹ unabhängig voneinander = H, substituiertes oder unsubstituiertes C₁- bis C₁₂-Alkyl, C₆-bis C₁₄-Aryl oder - (CH₂CH₂O)ₙH mit n = 1 bis 10;
- T =: C₁ bis C₁₂-Alkyl, C₆- bis C₁₄-Aryl, Halogen, NO₂, NR²₂, OR², CN, CO-R², CO-NR²₂, CO-OR², SR², SO₂R² oder SO₃R²,
wobei
die einzelnen Reste R² unabhängig voneinander = H, substituiertes oder unsubstituiertes C₁- bis C₁₂-Alkyl, C₆-bis C₁₄-Aryl oder -(CH₂CH₂O)ₙH mit n = 1 bis 10;
oder Y und T gemeinsam = -CO-O-CO- oder -CO-NR³-CO-,
wobei
- R³ =: H, substituiertes oder unsubstituiertes C₁- bis C₁₂-Alkyl, C₆- bis C₁₄-Aryl oder -(CH₂CH₂O)ₙH mit n = 1 bis 10;
- U =: C₁ bis C₁₂-Alkylenoxy, CO-NR⁴-, CO-O oder O,
wobei
R⁴ = H, substituiertes oder unsubstituiertes C₁- bis C₁₂-Alkyl oder C₆- bis C₁₄-Aryl;
- W =: m-fach substituiertes C₁- bis C₁₂-Alkylen, C₆- bis C₁₄-Arylen, C₈- bis C₁₆-Aralkylen oder
(-CH₂CH₂OCH₂CH₂-)ₙ mit n = 1 bis 10,
wobei vorstehende Reste noch weitere Substituenten haben können;
- m =: 2 bis 4 ; und
wobei
die bei den Resten R, R¹, R², R³ und R⁴ gegebenenfalls vorhandenen Substituenten und die bei W gegebenenfalls vorhandenen weiteren Substituenten COOH, OH, Halogen, C₁- bis C₁₂-Alkoxy, -N⁺-(C₁- bis C₁₂-Alkyl)₃, -O-P=O(OH)₂ oder -P=O(OH)₂ sind und wobei bei mehreren Substituenten an den Resten, diese Substituenten unabhängig voneinander gewählt werden können.

Dabei decken die obigen Formeln nur solche Verbindungen ab, die mit der Valenzlehre zu vereinbaren sind.

Weiter steht Formel (I) für die beiden Stellungsisomere 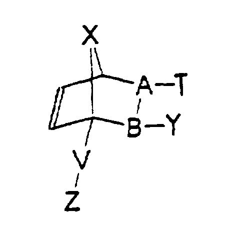 und 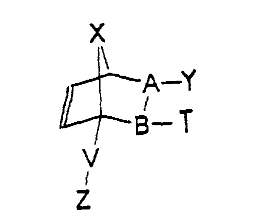 und Formel (II) für die beiden Stellungsisomere 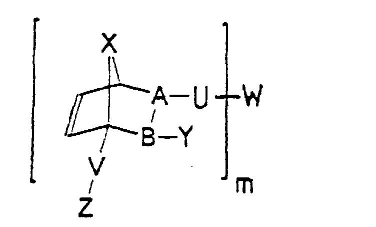 und

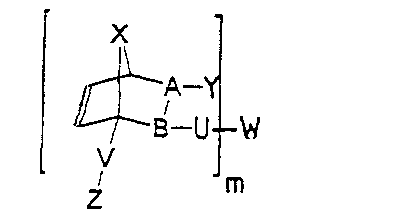

Dies gilt entsprechend auch für die weiteren in der Beschreibung und den Ansprüchen angegebenen Formeln, bei denen die in den Formeln (I) und (II) verwendete Form der Darstellung zur Erfassung der Stellungsisomere benutzt wird.

Ferner sind die Gruppen Y, T und U unabhängig voneinander in endo- oder exo-Stellung gebunden.

Typischerweise liegen die erfingungsgemäßen (Meth)acrylate in Form von Stereoisomerengemischen, insbesondere als Racemat, vor.

Die bei den Resten R, R¹, R², R³ und R⁴ gegebenenfalls vorhandenen Substituenten sind COOH, OH, Halogen, C₁- bis C₁₂-Alkoxy, -N⁺-(C₁- bis C₁₂-Alkyl)₃, -O-P=O(OH)₂ oder -P=O(OH)₂. Dies gilt auch für die bei W gegebenenfalls vorhandenen weiteren Substituenten. Sind mehrere Substituenten an den Resten vorhanden, so können diese unabhängig voneinander gewählt werden.

Im Falle der erfindungsgemäßen (Meth)acrylate, die unter die Formel (II) fallen, ist der Rest W m-fach, d.h. 2- bis 4-fach, mit dem in der Klammer angegebenen Norbornenyl-Rest substituiert. Darüber hinaus kann W auch noch mit den oben angegeben weiteren Substituenten substituiert sein.

Für bevorzugte unter die Formeln (I) und (II) fallende (Meth)acrylate sind zum besseren Verständnis nachstehend die entsprechenden Strukturformeln angegeben, wobei die Formeln auch für die entsprechenden Stellungsisomeren stehen, die sich durch Austausch der Substituenten an den Kohlenstoffatomen A und B ergeben.

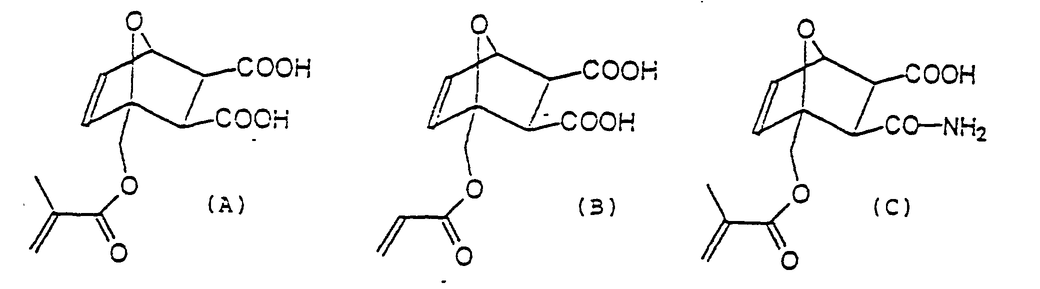

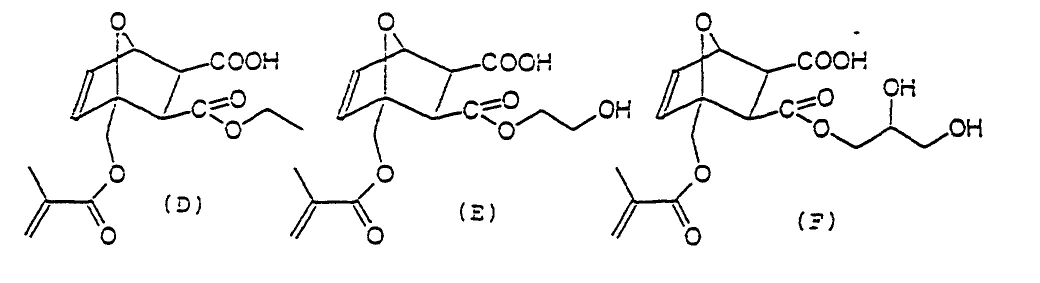

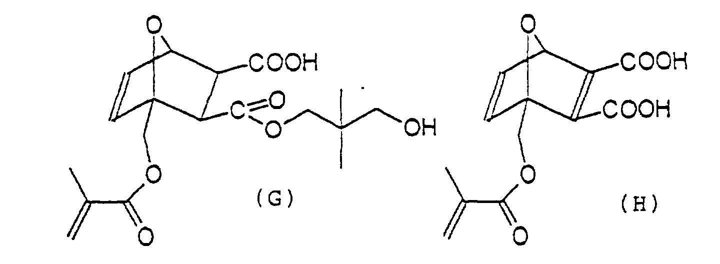

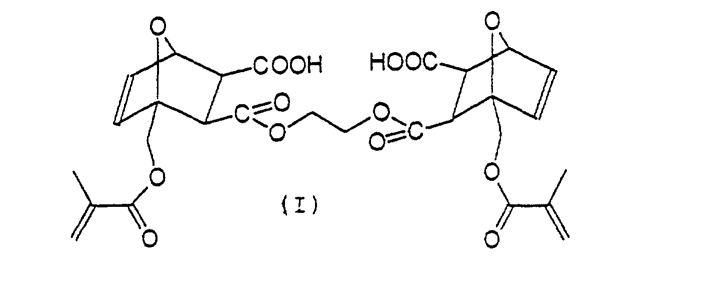

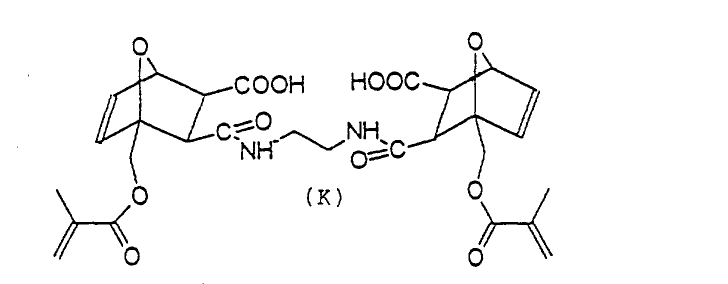

Weiter existieren für die oben angegeben Variablen der Formeln (I) und (II) unabhängig voneinander wählbare bevorzugte Definitionen, und diese sind wie folgt:
- A-B =: C-C oder C=C;
- X =: O;
- Z =: CH₂=CH-CO- oder CH₂=C(CH₃)-CO-;
- V =: CH₂-O;
- Y =: H, OH, COOH, CO-NH₂ oder CO-OR¹;
- R¹ =: H, substituiertes oder unsubstituiertes C₁- bis C₁₂-Alkyl oder C₆- bis C₁₄-Aryl;
- T =: OH, COOH, CO-NH₂ oder CO-OR²;
- R² =: H, substituiertes oder unsubstituiertes C₁- bis C₁₂-Alkyl oder C₆- bis C₁₄-Aryl;
oder Y und T gemeinsam = -CO-O-CO- oder CO-NR³-CO-;
- R³ =: H, substituiertes oder unsubstituiertes C₁- bis C₁₂-Alkyl oder C6- bis C₁₄-Aryl;
- U =: CO-O oder CO-NR⁴;
- R⁴ =: H oder C₁- bis C₅-Alkyl;
- W =: m-fach substituiertes C₁- bis C₁₂-Alkylen, welches noch weitere Substituenten haben kann; und/oder
- m =: 2.

Bevorzugte Verbindungen sind demgemäß solche, bei denen mindestens eine der Variablen der Formeln (I) und (II) die vorstehend beschriebene bevorzugte Definition aufweist.

Besonders bevorzugte erfindungsgemäße bicyclische (Meth)acrylate sind solche, bei denen mindestens eine der nachstehenden Definitionen (1.) bis (6.) erfüllt ist:
(1.) Bicyclische (Meth)acrylate der Formel (I) oder (II), bei denen
   - A-B =: C-C oder C=C,
   - X =: O und
   - V =: CH₂O.
(2.) Bicyclische (Meth)acrylate der Formel (I) oder (II), bei denen
   - Z =: CH₂=C(CH₃)-CO-.
(3.) Bicyclische (Meth)acrylate der Formel (I), bei denen
   - Y und T gemeinsam =: -CO-O-CO-
   oder
   - Y =: COOH und T = COOH.
(4.) Bicyclische (Meth)acrylate der Formel (I), bei denen
   - T =: COOH und
   - Y =: COOR¹,
   wobei R¹ = mit einer oder zwei OH-Gruppen substituiertes C₁- bis C₆-Alkyl.
(5.) Bicyclische (Meth)acrylate der Formel (II), bei denen
   - Y =: COOH,
   - U =: CO-O und
   - m =: 2.
(6.) Bicyclische (Meth)acrylate der Formel (II), bei denen
   - W =: m-fach substituiertes C₁- bis C₁₂-Alkylen, das mindestens eine OH- oder mindestens eine COOH-Gruppe als weitere Substituenten tragen kann.

Die erfindungsgemäßen funktionalisierten (Meth)acrylate der Formel (I) werden hergestellt, indem die substituierte Dien(meth)acryl-Verbindung (III) mit dem substituierten Dienophil (IV) im Rahmen einer Diels-Alder-Reaktion (vgl. H. Wollweber, Diels-Alder-Reaktion, G. Thieme-Verlag 1972) umgesetzt wird. 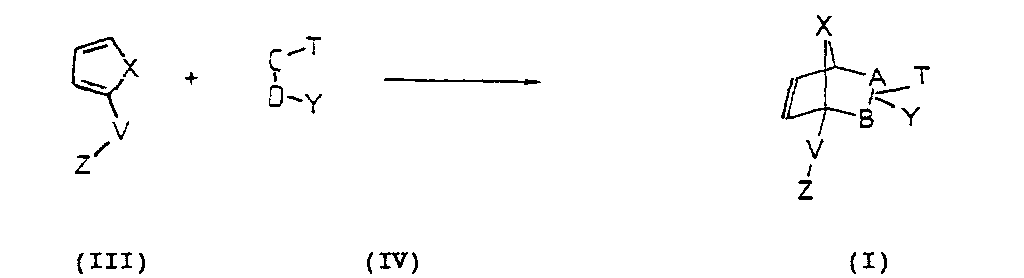 mit C-D = C=C oder C≡C

Die eingesetzte substituierte Dien(meth)acryl-Verbindung (III) ist allgemein durch Umsetzung von geeignet substituierten Furanen (X = O) oder Cyclopentadienen (X = CH₂) mit einer entsprechenden (Meth)acrylverbindung Z-OH oder Z-Cl zugänglich:

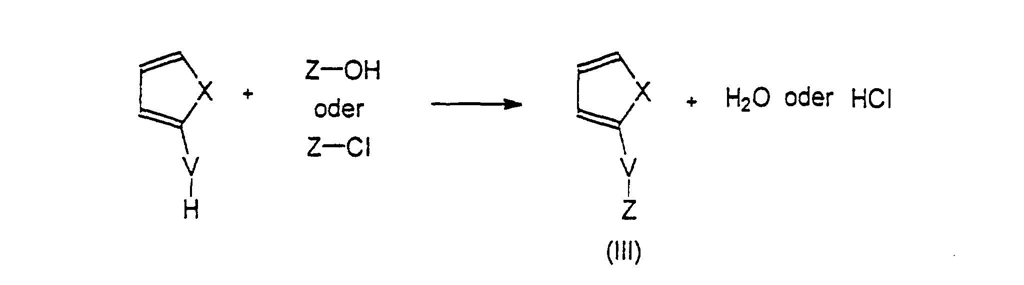

So kann die Dien(meth)acryl-Verbindung (III) z.B. durch Veresterung eines entsprechend substituierten Diens, wie käuflichem Furfurylalkohol oder Furfurylamin, mit (Meth)acrylsäure oder (Meth)acrylsäurechlorid hergestellt werden. Ein konretes Beispiel für eine solche Veresterung illustriert das nachstehende Reaktionsschema:

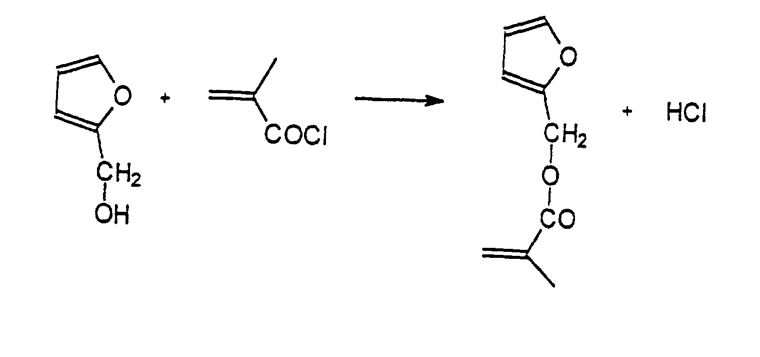

Analoge Umsetzungen sind mit geeignet substituierten Cyclopentadienen durchführbar.

Als Dienophile (IV) eignen sich vor allem Maleinsäure oder Acetylendicarbonsäure oder entsprechende Derivate wie z.B. Maleinsäureanhydrid oder Acetylendicarbonsäuredimethylester.

Zur Herstellung der (Meth)acrylate gemäß Formel (II) wird allgemein von einer bicyclischen Verbindung der Formel (V) ausgegangen, welche durch Diels-Alder-Reaktion aus entsprechenden Edukten erhältlich ist. Diese bicyclische Verbindung (V) wird dann mit einer Polyhydroxyverbindung der allgemeinen Formel (VI) unter Wasserabspaltung zum gewünschten (Meth)acrylat (II) kondensiert. Diese Umsetzung veranschaulicht die nachstehende Reaktionsgleichung.

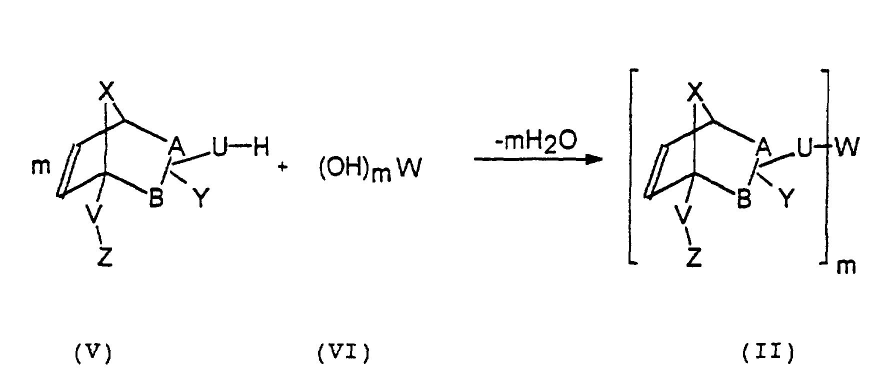

Spezielle und bevorzugte (Meth)acrylate der Formel (II) können unter Verwendung von (Meth)acrylaten der Formel (I) als Ausgangsmaterial hergestellt werden.

So ist es möglich, durch Kondensation eines (Meth)acrylats gemäß Formel (I), bei dem Y und T gemeinsam -CO-O-CO- bedeuten, mit einer mehrfunktionellen Verbindung mit mindestens m geeigneten reaktiven Gruppen, wie Hydroxy- oder Aminogruppen, z.B. einem Polyol, einem Polyamin oder einem Aminoalkohol, und insbesondere einer Polyhydroxyverbindung der Formel (VI), die entsprechenden erfindungsgemäßen (Meth)acrylate gemäß Formel (II) herzustellen.

Dabei kann die mehrfunktionale Verbindung neben den reaktiven Gruppen auch noch weitere Substituenten, wie z.B. NO₂ oder C=O aufweisen, wie oben bei der Definition von W bereits erwähnt wurde.

Die Kondensationsreaktion läßt sich unter den für die Alkoholyse und Aminolyse von Carbonsäureanhydriden allgemein bekannten Reaktionsbedingungen durchführen (vgl. Autorenkollektiv, Organikum Deutscher Verlag der Wissenschaften, Berlin 1973, S. 444 ff. und 453 ff.). Ein allgemeines sowie ein konkretes Beispiel für diese Herstellungsweise von bevorzugten erfindungsgemäßen (Meth)acrylaten der Formel (II) sind nachstehend anhand von Reaktionsgleichungen wiedergegeben.

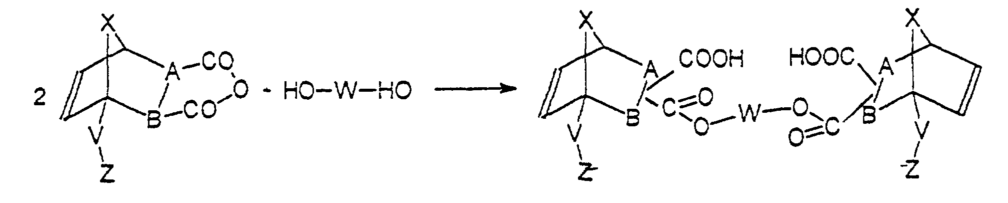

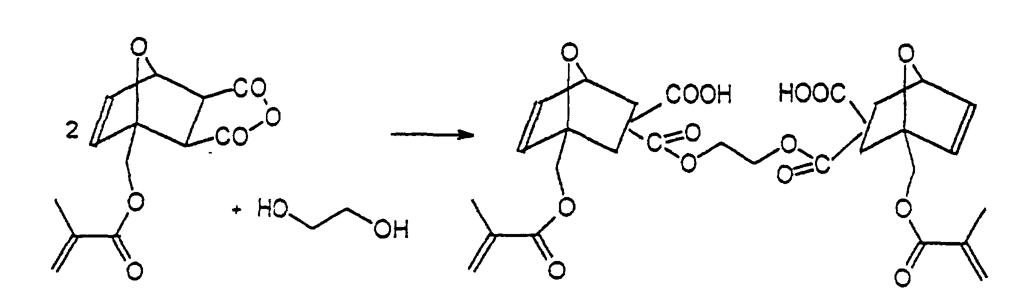

Beispiel für die als mehrfunktionelle Verbindungen einsetzbaren Verbindungen sind:

| | |
|---|---|
| Polyole | Ethylenglykol, Butandiol, Hexandiol, Glycerin, Di-oder Triethylenglycol. |
| Polyamine | 1,3-Diaminopropan, 1,6-Diaminohexan oder Phenylendiamin. |
| Aminoalkohol | Ethanolamin, Aminopropanol oder Triethanolamin. |

Aufgrund des Vorliegens von polymerisierbaren Gruppen eignen sich die erfindungsgemäßen bicyclischen (Meth)acrylate als Ausgangsmaterialien für die Herstellung von Polymeren und Copolymeren. Dabei lassen sie sich mit den bekannten Methoden der radikalischen oder anionischen Polymerisation homopolymerisieren oder zum Beispiel mit geeigneten Vinyl- oder (Meth)acrylmonomeren copolymerisieren. Vor der radikalischen Polymerisation kann, sofern gewünscht, durch ringöffnende Metathesepolymerisation zum Beispiel mit käuflichem Ruthenium(III)-chlorid in wäßrig-alkoholischer Lösung (vgl. S. Y. Lu et al., Makromol. Chem. Phys. 195, (1994) 1273) ein radikalisch vernetzbares Polymer hergestellt werden.

Neben den polymerisierbaren Gruppen können die erfindungsgemäßen (Meth)acrylate mehrere verschiedene funktionelle Gruppen, wie z.B. OH oder COOH enthalten. Derartige funktionelle Gruppen sind bei Verwendung der (Meth)acrylate in Adhäsiven vorteilhaft, da sie zur Erhöhung der Haftung der Adhäsive an verschiedenen Substraten, wie z.B. Kunststoff oder Metall, beitragen können.

Die erfindungsgemäßen (Meth)acrylate können als Bestandteil von Adhäsiven, wie technischen Adhäsiven zum Beispiel für Herstellung von Metall-Kunststoff-Verbunden, eingesetzt werden.

Bevorzugt werden die erfindungsgemäßen bicyclischen (Meth)acrylate als Dentalmaterial, wie Primerkomponente von Dentinadhäsiven, oder Bestandteil von Dentalmaterialien, wie z.B. Dentalkompositen, Fissurenversieglern, Materialien zur Desensibilisierung von Dentin, oder Dentinadhäsiven eingesetzt, wobei sich ihre Fähigkeit als vorteilhaft erweist, daß sie sich bei Raumtemperatur radikalisch härten lassen.

Bei Verwendung der erfindungsgemäßen (Meth)acrylate als Bestandteil von Dentalmaterialien werden sie in einer Menge von 0,1 bis 60, insbesondere 5 bis 45 Gew.-%, bezogen auf das Dentalmaterial, eingesetzt. Zur Herstellung der Dentalmaterialien werden die erfindungsgemäßen (Meth)acrylate insbesondere mit polymerisierbaren organischen Bindemitteln, Füllstoffen, Polymerisationsinitiatoren und/oder weiteren Zusätzen, wie üblichen Stabilisatoren, z.B. Hydrochinonmonomethylether (MEHQ) oder 2,6-Di-tert.-butyl-4-methylphenol (BHT), UV-Absorbern, Pigmenten, Farbstoffen oder Lösungsmitteln kombiniert.

Als polymerisierbare organische Bindemittel eignen sich alle für einen Dentalwerkstoff brauchbaren Bindemittel, insbesondere monofunktionelle oder polyfunktionelle (Meth)acrylate, die allein oder in Mischungen eingesetzt werden können. Bevorzugte Beispiele für diese Verbindungen sind Methylmethacrylat, Isobutylmethacrylat, Cyclohexylmethacrylat, Tetraethylenglycoldimethacrylat, Triethylenglycoldimethacrylat, Diethylenglycoldimethyacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4-(3-methacryloxy-2-hydroxypropoxy)-phenylpropan (Bis-GMA) sowie die Produkte der Reaktion von Isocyanaten, insbesondere Di- und/oder Triisocyanaten, mit OH-gruppenhaltigen Methacrylaten. Besonders bevorzugte Beispiele für die zuletzt genannten Produkte sind durch Reaktion von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat erhältlich.

Die organischen Bindemittel werden üblicherweise in einer Menge von 0,1 bis 60 Gew.-% in dem erfindungsgemäßen Dentalmaterial eingesetzt.

Beispiele für bevorzugte Füllstoffe sind Quarz-, Glaskeramik- und Glaspulver, insbesondere Bariumsilikatgläser, Li/Al Silikatgläser und Bariumgläser, Aluminium- oder Siliciumoxide, feinstteilige Kieselsäuren, insbesondere pyrogene oder gefällte Kieselsäuren, röntgenopake Füllstoffe, wie Ytterbiumtrifluorid.

Die Füllstoffe werden typischerweise in einer Menge von 0 bis 80 Gew.-%, bezogen auf das Dentalmaterial, eingesetzt.

Die erfindungsgemäßen Dentalmaterialien können heiß, kalt oder durch Licht polymerisiert werden. Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden. Darüber hinaus sind auch 2,2'-Azoisobuttersäurenitril (AIBN), Benzpinakol und 2,2'-Dialkylbenzpinakole geeignet.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet. Darüber hinaus eignet sich auch die Gruppe der Acylphosphinoxide gut zur Initiierung der Photopolymerisation. Zur Beschleunigung der Initiierung werden die Photoinitiatoren vorzugsweise zusammen mit einem Reduktionsmittel, besonders bevorzugt mit einem Amin insbesondere einem aromatischen Amin eingesetzt.

Als Initiatoren für die Kaltpolymerisation werden Radikale liefernde Redox-Systeme, zum Beispiel Benzoyl- bzw. Lauroylperoxid zusammen mit Aminen wie N,N-Dimethyl-p-toluidin, N,N-Dihydroxyethyl-p-toluidin oder anderen strukturverwandten Aminen eingesetzt.

Speziell bei Dentalmaterialien zur Zementierung von Dentalrestaurationen, wie Glaskeramik-Inlays, -Onlays, -Teilkronen und - Kronen, hat sich die Kombination von Photoinitiatoren mit unterschiedlichen Redoxsystemen bewährt. Bevorzugt sind Kombinationen aus Campherchinon, Benzoylperoxid und Aminen wie N,N-Dimethyl-p-toluidin und/oder N,N-Cyanoethylmethylanilin.

Die Konzentration der Initiatoren liegt bevorzugt im Bereich von 0,05 bis 2,0 Gew.-%, besonders bevorzugt im Bereich von 0,1 bis 0,8 Gew.-%, bezogen auf das Dentalmaterial.

Besonders bevorzugt werden die erfindungsgemäßen bicyclischen (Meth)acrylate als Bestandteil von Dentinadhäsiven eingesetzt. Überraschenderweise verleihen sie dabei dem Dentinadhäsiv eine verbesserte Haftung am Dentin, ohne daß im Vergleich zu bekannten Materialien weitere Filmbildner notwendig wären. Diese verbesserte Haftungswirkung wird auf das gemeinsame Vorliegen von polymerisierbaren Gruppen und funktionellen Gruppen in den erfindungsgemäßen Verbindungen zurückgeführt. Es wird angenommen, daß zum Beispiel bei der Verwendung von Dentinadhäsiven auf der Grundlage der erfindungsgemäßen (Meth)acrylate zum Befestigen eines Dentalkomposits einerseits die (Meth)acrylatgruppe eine sichere Verbindung mit dem Kompositmaterial gewährleistet und andererseits die funktionellen Gruppen, wie zum Beispiel Carboxylgruppen, über Wechselwirkungen mit der Zahnhartsubstanz zu einem Verbund mit diesem führen. Darüber hinaus ist auch die Ausbildung kovalenter Bindungen, zum Beispiel zwischen den NH-Gruppen des Dentinkollagens und den ungesättigten bicyclischen Gruppen der erfindungsgemäßen (Meth)acrylate, möglich.

Die Erfindung wird im folgenden anhand von Beispielen näher erläutert.

### Beispiele

### Beispiel 1: exo-1-[(Methacryloyloxy)methyl]-7-oxabicyclo[2.2.1]hept-5-en-2,3-dicarbonsäureanhydrid (1)

In einen 500 ml Zweihalskolben mit mechanischem Rührwerk, Thermometer und CaCl₂-Rohr wurden 166 g (1 mol) Furfurylmethacrylat, 107,8 g (1,1 mol) Maleinsäureanhydrid, 250 ml Butylacetat und 0,01 g Hydrochinonmonomethylether (MEHQ) gegeben, und die erhaltene Mischung wurde 2 Tage bei Raumtemperatur gerührt. Der ausgefallene Feststoff wurde abgesaugt, mit 100 ml Butylacetat gewaschen und im Vakuum bis zur Gewichtskonstanz getrocknet. Es fielen ca. 140 g Feststoff an. Die Mutterlauge wurde weiter gerührt, und nach 21 Tagen wurde zusätzliches Produkt wie oben beschrieben isoliert (ca. 60 g).
- Ausbeute:: ca. 200 g (76 %) Schmp.: 109 - 110 °C (farblose Kristalle)

| | | | | |
|---|---|---|---|---|
| Elementaranalyse | C₁₃H₁₂O_{6:} | Ber. | C 59,08 | H 4,58 |
| | (264,23) | Gef. | C 58,89 | H 4,61 |

- ¹H-NMR(90 MHz, CDCl₃):: 1,91 (s, 3H, CH₃); 3,42 und 3,52 (d, 2× 1H, H-2,3); 4,55 und 4,83 (d, 2×1H, CH₂O); 5,33 (s, 1H, H-4); 5,67 und 6,04 (s, 2×1H, CH₂=); 6,52 (d, 1H, H-6); 6,64 (s, 1H, H-5) Zuordnung mit COSY
- ¹³C-NMR (75 MHz, CDCl₃):: 17,99 (CH₃↑); 50,07 und 51,82 (C-2,3↑); 61,00 (CH₂O↓); 81,69 (C-4↑); 89,97 (C-1(-)); 126,17 (CH₂=↓); 135,36 (CH₂=C); 137,06 und 137,72 (C-5,6↑); 165,89, 169,39 und 170,77 (alle C=O)

### Beispiel 2: exo-1-[(Methacryloyloxy)methyl]-7-oxabicyclo[2.2.1]hept-5-en-2,3-dicarbonsäure (2)

In einem 100 ml Zweihalskolben mit Tropftrichter, Magnetrührwerk und Thermometer wurden 0,74 g (0,041 mol) Wasser und 0,01 g Phenothiazin in 50 ml Tetrahydrofuran (THF) gelöst, und es wurden zu dieser Lösung 10,6 g (0,04 mol) (1) hinzugefügt, welches gemäß Beispiel 1 hergestellt worden war. Es wurde auf 5 - 10 °C gekühlt und unter Rühren eine Lösung von 8,0 g (0,08 mol) Triethylamin (TEA) und 0,25 g 4-Dimethylaminopyridin (DMAP) in 15 ml THF langsam zugetropft. Dann wurde noch 2 Stunden bei Raumtemperatur nachgerührt, und die entstandene Reaktionsmischung wurde auf 115 ml 2n HCl (pH = 1-2) gegossen. Die wäßrige Lösung wurde abgetrennt und mit 3 x je 100 ml Ether extrahiert, welcher mit 2,6-Di-tert.-butyl-4-methylphenol (BHT) stabilisiert war. Die vereinigten organischen Extrakte wurden über Natriumsulfat getrocknet, und das Lösungsmittel wurde abdestilliert. Es blieb ein Öl zurück, welches kurz im Feinvakuum getrocknet wurde. Der daraufhin gebildete schmierige Feststoff (8,5 g) wurde mit wenig Butylacetat 3 Stunden lang gut verrührt. Der Feststoff wurde dann abgesaugt, mit etwas Butylacetat gewaschen und im Feinvakuum bis zur Gewichtskonstanz getrocknet.
- Ausbeute:: 6,1 g (54 %) Schmp.: 118 - 120 °C (Zersetzung, farblose Kristalle)

| | | | | |
|---|---|---|---|---|
| Elementaranalyse | C₁₃H₁₄O₇ | Ber. | C 55,32 | H 5,00 |
| | (282,24) | Gef. | C 55,46 | H 5,14 |

- ¹H-NMR(300MHz, DMSO-d₆):: 1,86 (s, 3H, CH₃; 2,82 (d, 2H, H-2,3); 4,52 (q, 2H, CH₂O); 5,13 (s, 1H, H-4); 5,67 und 6,00 (s, 2×1H, CH₂=); 6,38 (d, 1H, H-6); 6,50 (m, 1H, H-5); 12,35 (b, 2H, COOH)
- ¹³C-NMR (75 MHz, CDCl₃):: 17,86 (CH₃↑); 47,96 und 48,85 (C-2,3↑); 61,88 (CH₂O↓); 79,45 (C-4↑); 88,61 (C-1(-)); 126,31 (CH₂=↓); 135,48 (CH₂=C(-)); 136,58 und 137,59 (C-5,6t); 166,16, 171,81 und 172,20 (alle C=O(-)).

### Beispiel 3: exo-1-[(Methacryloyloxy)methyl]-7-oxabicyclo[2.2.1]hept-5-en-2,3-dicarbonsäuremono(2-hydroxyethyl)ester (3)

In einem 100 ml Zweihalskolben mit Tropftrichter, CaCl₂-Rohr und Thermometer wurden 2,6 g (0,041 mol) Ethylenglykol und 0,01 g Phenothiazin in 50 ml THF gelöst. In dieser Lösung wurden dann 10,6 g (0,04 mol) (1) suspendiert. Es wurde auf ca. - 15 °C gekühlt, und dann wurde unter Rühren eine Lösung von 4,0 g (0,04 mol) TEA und 0,25 g (2 mmol) DMAP in 10 ml THF innerhalb von 45 Minuten zugetropft. Man ließ auf Raumtemperatur erwärmen und rührte zur Vervollständigung der Reaktion noch 5 Stunden nach. Das Reaktionsgemisch wurde in einer Mischung von 100 ml 10 %iger wäßriger NaHCO₃ Lösung und 50 ml Ether aufgenommen. Die wäßrige Phase wurde abgetrennt, nochmals mit 50 ml Ether gewaschen und dann mit ca. 10 ml konzentrierter Salzsäure auf einen pH = 1-2 eingestellt. Dabei fiel ein schmieriges Öl an, das in 300 ml Ether aufgenommen und mit 0,01 g BHT stabilisiert wurde. Die Ether-Lösung wurde gegebenenfalls durch Filtration von unlöslichen Materialien befreit. Die wäßrige Phase wurde nochmals mit 2 x je 100 ml Ether extrahiert, und die vereinigten organischen Extrakte wurden über Na₂SO₄ getrocknet. Nach Abdestillieren des Lösungsmittels im Vakuum bei maximal 30 °C wurde das erhaltene Produkt im Feinvakuum getrocknet.
- Ausbeute:: 6,1 g (51 %), farbloses schaumig-klebriges Produkt

| | | | | |
|---|---|---|---|---|
| Elementaranalyse | C₁₅H₁₈O₈ | Ber. | C 55,21 | H 5,56 |
| | (326,30) | Gef. | C 55,45 | H 5,63 |

- ¹H-NMR(90 MHz, DMSO-d₆):: 1,87 (s, 3H, CH₃); 2,96 (s, 2H, CHCOO); 3,4-3,7 (m, 2H, CH₂OH); 3,95-4,3 (m, 2H, H-2,3); 4,64 (d, 2H, CH₂-Methacryl); 5,27 (s, 1H, H-4); 5,78 und 6,10 (s, 2×1H, CH₂=); 6,42 - 6,68 (m, 2H, H-5,6); 7,8 (s,b, 2 H, OH H/D-Austausch, Verunreinigung: Ether)

### Beispiel 4: 1,2-Bis[exo-1-[(methacryloyloxy)methyl]-7-oxabicyclo[2.2.1]hept-5-en-2(3)-carbonsäure-3(2)-carbonyloxy]ethan(4)

In einem 100 ml Zweihalskolben mit Tropftrichter, CaCl₂-Rohr und Thermometer wurden 1,25 g (0,02 mol) Ethylenglykol und 0,01 g Phenothiazin in 50 ml THF gelöst. Zu dieser Lösung wurden 10,6 g (0,04 mol) (1) gegeben. Es wurde auf ca. - 15 °C gekühlt, und dann wurde unter Rühren eine Lösung von 4,0 g (0,04 mol) TEA und 0,25 g (2 mmol) DMAP in 10 ml THF zugetropft. Man ließ auf Raumtemperatur erwärmen und rührte zur Vervollständigung der Reaktion noch 3,5 Stunden nach. Das Reaktionsgemisch wurde mit 60 ml 2n HCl schwach sauer eingestellt, woraufhin sich zwei Phasen bildeten. Die organische Phase wurde in 100 ml Ether aufgenommen und die wäßrige Phase wurde nochmals mit 100 ml Ether extrahiert. Die vereinigten organischen Phasen wurden dann zweimal mit je 100 ml 10 %iger wäßriger NaHCO₃-Lösung ausgeschüttelt. Die vereinigten Bicarbonatlösungen wurden mit ca. 10 ml konzentrierter Salzsäure auf pH = 1-2 eingestellt. Dabei fiel ein schmieriges Öl an, das in 400 ml Ether und 100 ml Methylenchlorid aufgenommen und mit 0,01 g BHT stabilisiert wurde. Die erhaltene Lösung wurde gegebenenfalls durch Filtration von unlöslichem Material befreit und über Na₂SO₄ getrocknet. Anschließend wurde das Lösungsmittel im Vakuum bei maximal 30 °C abdestilliert, und das erhaltene Produkt wurde im Feinvakuum getrocknet.
- Ausbeute:: 8,0 g (68 %) hochviskoses Harz

| | | | | |
|---|---|---|---|---|
| Elementaranalyse | C₂₈H₃₀O₁₄ | Ber. | C 56,95 | H 5,12 |
| | (590,53) | Gef. | C 56,86 | H 5,31 |

- ¹H-NMR(300 MHz, DMSO-d₆):: 1,87 (s, 6H, CH₃); 2,96 (m, 4H, CHCOO); 3,95 - 4,3 (m, 4H, H-2,3); 4,4 - 4,6 (m, 4H, CH₂-Furyl); 5,27 (m, 2H, H-4); 5,68 und 6,02 (s, 2×1H, CH₂=); 6,3 - 6,6 (m, 4H, H-5,6); 12,5 (s, b, COOH), (Verunreinigung: Ether).

### Beispiel 5: exo-1-[(Methacryloyloxy)methyl]-7-oxabicyclo[2.2.1]hept-5-en-2,3-di-carbonsäuremono(2,3-dihydroxypropyl)ester (5)

Analog zu Beispiel 3 erfolgten die Umsetzung von 11,3 g (0,12 mol) wasserfreiem Glycerin mit 10,6 g (0,04 mol) (1) in Gegenwart von 0,25 g DMAP, 4,0 g TEA und 0,01 g Phenothiazin in 80 mol THF sowie die Abtrennung des erhaltenen Produktes (5).
- Ausbeute:: 1,33 g (9 %) (viskoses Öl)

| | | | | |
|---|---|---|---|---|
| Elementaranalyse | C₁₆H₂₀O₉ | Ber. | C 53,93 | H 5,66 |
| | (356,32) | Gef. | C 54,22 | H 5,67 |

- ¹H-NMR(90 MHz, CDCl₃):: 1,98 (s, 3 H, CH₃); 3,08 (s, 2 H, H-2,3); 3,5 - 4,45 (m, 5 H, CH₂CHCH₂); 4,7 (s, 2 H, CH₂-norbornyl), 5,47 (s, 1 H, H-4); 5,67 und 6,18 (s, 2 × 1 H, CH₂=); 6,35 - 6,95 (m, b, 5 H, H-5,6 + OH, H/D-Austausch).
- ¹³C-NMR(75 MHz, CDCl₃):: 18,25 (CH₃-C=CH₂); 21,57 (C(CH₃)₂); 35,35 (C(CH₃)₂); 48,9 und 49,8 (C-2,3); 61,9 (COOCH₂-norbornyl); 63,1 (CH-OH); 65,8 und 69,9 (CH₂O-propyl); 128,9 (C=CH₂); 135,5 und 137,5 (C-5,6); 166,7, 170,8 und 173,9 (alle C=O).

### Beispiel 6: exo-1-[(Methacryloyloxy)methyl]-7-oxabicyclo[2.2.1]hept-5-en-2,3-di-carbonsäuremono(3-hydroxy-2,2-dimethyl-propyl)ester (6)

Analog zu Beispiel 3 erfolgten die Umsetzung 12,8 g (0,12 mol) wasserfreiem 2,2-Dimethyl-1,3-propandiol mit 10,6 g (0,04 mol) (1) in Gegenwart von 0,25 g DMAP, 4,0 g TEA und 0,01 g Phenothiazin in 80 ml THF sowie die Abtrennung des Produktes (6). Das erhaltene ölige Rohprodukt wurde zusätzlich bei 0°C 1 Stunde lang mit Butylacetat gut verrührt. Der erhaltene Niederschlag wurde dann abgesaugt und am Rotationsverdampfer 7 Stunden lang bei 25°C (20 mbar) getrocknet.
- Ausbeute:: 3,0 g (20 %), Schmp.: 108°C (farblose Kristalle)

| | | | | |
|---|---|---|---|---|
| Elementaranalyse | C₁₈H₂₄O₈ | Ber. | C 58,69 | H 6,57 |
| | (368,38) | Gef. | C 58,71 | H 6,53 |

- ¹H-NMR (300 MHz, CDCl₃):: 0,85 und 0,89 (s, 2 × 3 H, CH₃-propyl); 1,94 (s, 3 H, CH₃-C=CH₂); 2,90 und 3,05 (d, 2 × 1 H, H-2,3); 3,30 und 3,40 (d, 2 × 1 H, CH₂OH); 3,78 und 3,89 (d, 2 × 1 H), 4,57 und 4,77 (d, 2 × 1 H, COOCH₂-norbornyl); 5,46 (s, 1 H, H-4); 5,60 und 6,14 (s, 2 × 1 H, CH₂=); 6,41 und 6,51 (d, 2 × 1 H, H-5,6); 8,0 (s, b, 2 H, OH + COOH).
- ¹³C-NMR (75 MHz, CDCl₃):: 18,2 (CH₃-C=CH₂); 21,6 (C(CH₃)₂); 35,4 (C(CH₃)₂); 48,9 und 49,6 (C-2,3); 61,9 (COOCH₂-norbornyl); 66,9 und 69,6 (CH₂O-propyl); 79,9 (C-4); 89,3 (C-1); 126,6 (C=CH₂); 135,5 (C=CH₂); 137,2 und 137,3 (C-5,6); 166,8, 171,2 und 174,6 (alle C=O).

### Beispiel 7: exo-1-[Methacryloyloxy)methyl]-7-oxabicyclo[2.2.1]hept-5-en-2,3-di-carbonsäuremono(8-hvdroxy-3,6-dioxaoctyl)ester (7)

Analog zu Beispiel 3 erfolgten die Umsetzung 6,2 g (0,041 mol) wasserfreiem Triethylenglykol mit 10,6 g (0,04 mol) (1) in Gegenwart von 0,25 g DMAP, 4,0 g TEA und 0,01 g Phenothiazin in 60 ml THF sowie die Abtrennung des Produktes (7).
- Ausbeute:: 3,23 g (20 %) schwach gelbliches, viskoses Öl x

| | | | | |
|---|---|---|---|---|
| Elementaranalyse | C₁₉H₂₆O₁₀ | Ber. | C 55,07 | H 6,32 |
| | (414,40) | Gef. | C 55,22 | H 6,52 |

- ¹H-NMR (90 MHz, CDCl₃):: 1,23 (Verunreinigung, Ether); 1,98 (s, 3 H, CH₃); 2,9 - 3,1 (m, 2 H, H-2,3); 3,55 - 3,9 (m, 10 H, OCH₂CH₂O); 4,1 - 4,5 (m, 2 H, COOCH₂CH₂); 4,55 - 4,95 (m, CH₂-norbornyl); 5,45 (d, 1 H, H-4); 5,66 und 6,18 (s, 2 × 1 H, CH₂=); 6,4 - 6,68 (m, 2 H, H-5,6); 6,90 (s, 2 H, COOH + OH).

### Beispiel 8: 1-Methacryloyloxymethyl-7-oxabicyclo[2.2.1]hept-2,5-dien-2,3-di-carbonsäuredimethvlester (8)

In einem 250 ml Dreihalskolben mit Magnetrührer, Tropftrichter, Thermometer, CaCl₂-Rohr wurden 12,0 g (0,05 mol) 1-Hydroxymethyl-7-oxabicyclo[2.2.1]hept-2,5-dien-2,3-dicarbonsäuredimethylester, der einfach durch Diels-Alder-Umsetzung von Acetylendicarbonsäuredimethylester mit Furfurylalkohol zugänglich ist, 5,4 g (0,054 mol) TEA und 0,01 g BHT in 80 ml THF gelöst und bei 0°C unter Rühren vorgelegt. Dazu tropfte man eine Lösung von 5,9 g (0,056 mol) Methacrylsäurechlorid in 20 ml THF so langsam zu, daß die Temperatur zwischen 0 - 5 °C blieb. Man ließ das Reaktionsgemisch unter weiterem Rühren innerhalb 1 Stunde auf Raumtemperatur kommen. Das gebildete TEA-Hydrochlorid wurde abgesaugt und mit Diethylether gewaschen, und das Filtrat wurde mit 35 ml gesättigter Kochsalzlösung, die mit konzentrierten HCl auf pH 1 eingestellt war, extrahiert. Dann wurde die organische Phase mit 2 x 50 ml gesättigter Kochsalzlösung, die mit 10 ml gesättigter Soda-Lösung basisch eingestellt war, und abschließend mit 2 x 100 ml gesättigter Kochsalzlösung gewaschen. Zur Trocknung wurde die organische Phase 20 Minuten lang mit 50 g wasserfreiem Natriumsulfat gerührt. Es wurde abfiltriert und am Rotationsverdampfer bei 30°C im Vakuum eingeengt. Der erhaltene Rückstand wurde dann im Feinvakuum bis zur Gewichtskonstanz getrocknet. Das so erhaltene Produkt wurde mittels Säulenchromatographie (Kieselgel 60 (220-440 mesh)/Toluol) gereinigt.
- Ausbeute:: 8,8 g (57 %) (gelbliches Öl)

| | | | | |
|---|---|---|---|---|
| Elementaranalyse | C₁₅H₁₆O₇ | Ber. | C 58,44 | H 5,23 |
| | (308,29) | Gef. | C 58,50 | H 5,15 |

- ¹H-NMR (90 MHz, CDCl₃):: 1,98 (s, 3 H, CH₃-C=); 3,80 (s, 6 H, CH₃); 4,95 (s, 2H, CH₂); 5,67 (s, 1 H, H-1); 5,80 und 6,20 (s, 2 × 1 H, CH₂=); 7,1 - 7,45 (m, 2 × 1 H, H-5,6).

### Beispiel 9: Radikalische Lösungspolymerisation der erfindungsgemäßen funktionalisierten Norbornen(meth)acrylate (1), (2) und (3)

Durch Auflösen der jeweiligen erfindungsgemäßen (Meth)acrylate (1), (2) und (3) in Dimethylformamid (DMF) in einem Schlenkgefäß wurden Monomerlösungen mit einer Monomerkonzentration von 1,0 Mol/l hergestellt. In die jeweilige Lösung wurde Azobisisobutyronitril als Initiator in einer Menge gegeben, um eine Initiatorkonzentration von 0,02 Mol/l zu ergeben. Nach Eingabe eines mit Teflon beschichteten Magnetrührstabes wurden die Lösungen zum Entgasen in der üblichen Weise sekuriert, indem sie mehrfach unter Inertgas eingefroren und im Vakuum aufgetaut wurden, und anschließend unter Verwendung eines Schnellbelichtungstischgerätes SUNTEST CPS (Heraeus) bei 25°C in einem thermostatierten Bad unter Rühren mit UV-Licht bestrahlt. Die Polymerisation wurde nach 1 Stunde abgebrochen, indem das Reaktionsgemisch zur Ausfällung des Polymerisats mit der 10-fachen Menge Diethylether versetzt wurde. Das durch Filtration isolierte Polymer wurde dann im Feinvakuum bis zur Gewichtskonstanz getrocknet.

Der für das jeweilige Monomer bestimmte Monomerumsatz und die zahlenmittlere Molmasse der jeweilig erhaltenen Polymeren sind in der nachfolgenden Tabelle angegeben:

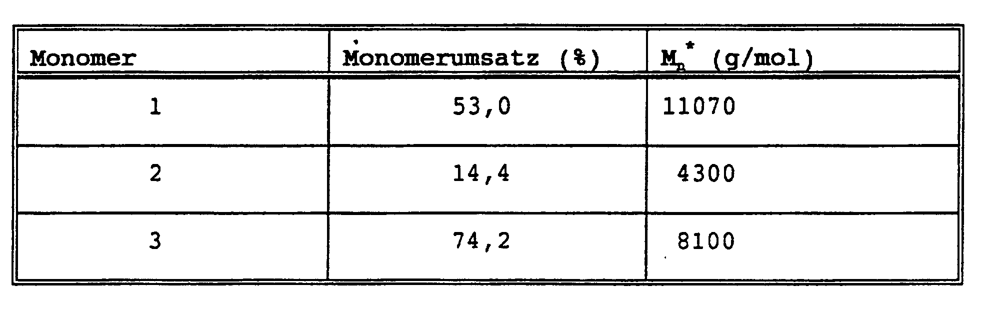

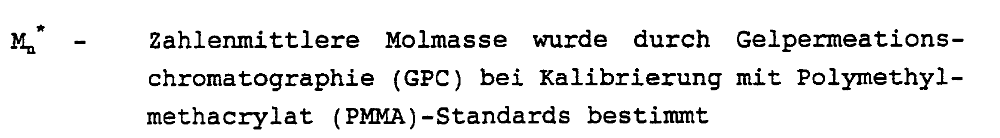

### Beispiele 10 bis 14: Erfindungsgemäße Primer und Dentinadhäsive und Vergleichsbeispiel

Dentinadhäsive bestehen in der Regel aus einer oberflächenmodifizierenden Komponente (Primer) und einer schichtbildenden Haftkomponente (Adhäsiv). Zusätzlich kann auch noch ein sogenanntes Bonding zur Verbesserung der Schichtbildung verwendet werden.

In den folgenden Beispielen 10 bis 13 werden erfindungsgemäße Primer-Formulierungen beschrieben, die das erfindungsgemäße funktionalisierte bicyclische (Meth)acrylat (2) oder (3) enthalten, sowie deren Verwendung als Komponente von erfindungsgemäßen Dentinadhäsiven. Die Herstellung dieser (Meth)acrylate ist in Beispiel 2 bzw. 3 beschrieben.

Das Beispiel 14 bezieht sich auf einen herkömmlichen Primer ohne Gehalt an den erfindungsgemäßen bicyclischen (Meth)acrylaten und es dient als Vergleichsbeispiel.

Die Beispiele 10 bis 13 zeigen, daß die erfindungsgemäßen Verbindungen als einziger funktioneller Bestandteil oder in Kombination mit weiteren Filmbildnern, wie z.B. Hydroxyethylmethacrylat (HEMA), eingesetzt werden können.

Dabei ist es möglich, daß die Formulierungen als 3-Schichtsystem oder zur Vereinfachung der Handhabung als 2-Schichtsystem verwendet werden. Um als 2-Schichtsystem einen guten Verbund zur Zahnhartsubstanz zu erzielen, werden noch weitere Filmbildner dem Primer oder dem Bonding zugegeben.

Die verwendeten Primer-Formulierungen wurden hergestellt, indem man die einzelnen Bestandteile unter Rühren in das vorgelegte Lösungsmittelgemisch gab und bis zum Erhalt einer homogenen, klaren Lösung weiterrührte.

Zur Ermittlung der mit einzelnen Primer-Formulierungen als Komponente von Dentinadhäsiven erzielbaren Scherhaftwerte wurden zuerst Dentinoberflächen von extrahierten, eingebetteten Zähnen mit 500er Schleifpapier plangeschliffen und mittels Druckluft getrocknet. Dann wurde wie nachstehend angegeben weiter verfahren:

### 2-Schichtsystem

(a) Auf diese Dentinoberflächen wurde dann die jeweilige Formulierung innerhalb von 30 Sekunden aufgetragen. Nach einer Einwirkzeit von 30 Sekunden wurde die Formulierung abgeblasen. Die freigelegte Oberfläche erschien feucht.
(b) Dann wurde ein lichthärtendes Bonding, oder ein modifiziertes Bonding aufgetragen und belichtet. Anschließend wurde eine teilbare Teflonform (d = 4 mm, h = 6 mm) mit einer Halterung auf der Dentinoberfläche fixiert, und es wurde ein lichthärtendes Füllungskomposit, nämlich Tetric (Vivadent Ets., Liechtenstein), in einem durch die Teflonform vorgegebenen Volumen und auf einer dadurch bestimmten Haftfläche schichtweise auf die Dentinoberfläche aufpolymerisiert. Die Scherhaftwerte wurden nach 24 Stunden Lagerung in Wasser bei 37 °C gemäß ISO-TR 11 405: Dental material - Guidance on testing of adhesion to tooth structure bestimmt.

### 3-Schichtsystem

Die 3-Schichtsysteme wurden wie die 2-Schichtsysteme hergestellt, wobei jedoch zwischen den Schritten (a) und (b) als schichtbildende Haftkomponente auch noch die Adhäsiv-Komponente eines Dentinadhäsivs, nämlich eine Mischung aus 35 Gew.-% Tetraethylenglycoldimethacrylat, 5 Gew.-% Glutaraldehyd, und 60 Gew.-% Wasser, auf die Oberfläche aufgetragen und mit einem Luftbläser sanft verblasen und damit verteilt wurde, woraufhin ein dünner Film auf der Dentinoberfläche erschien.

Die Zusammensetzung des eingesetzten Bondings und des verwendeten modifizierten Bondings waren wie folgt:

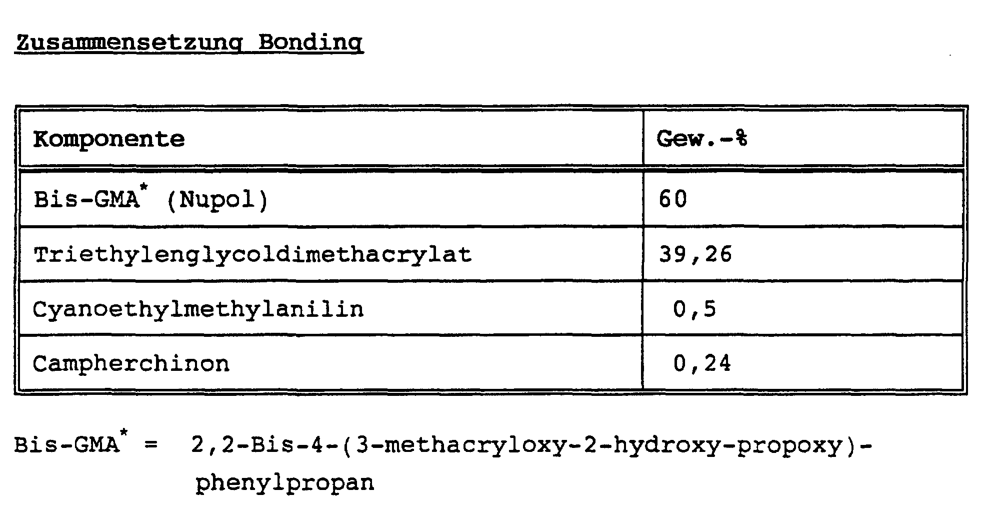

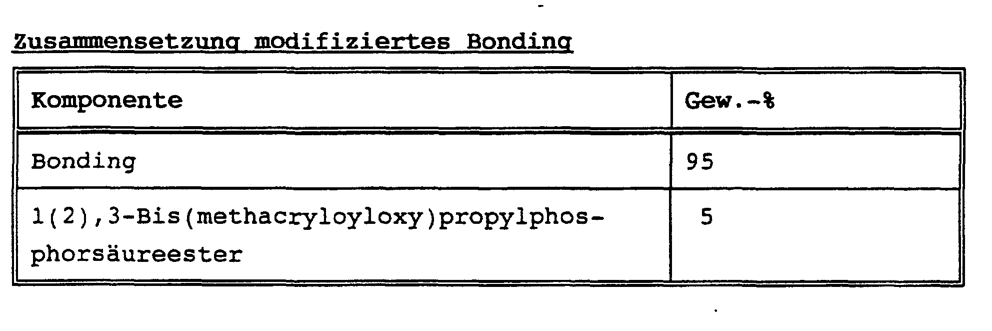

### Beispiel 10: Formulierungen mit (Meth)acrylat (3) als einzigem funktionellen Bestandteil

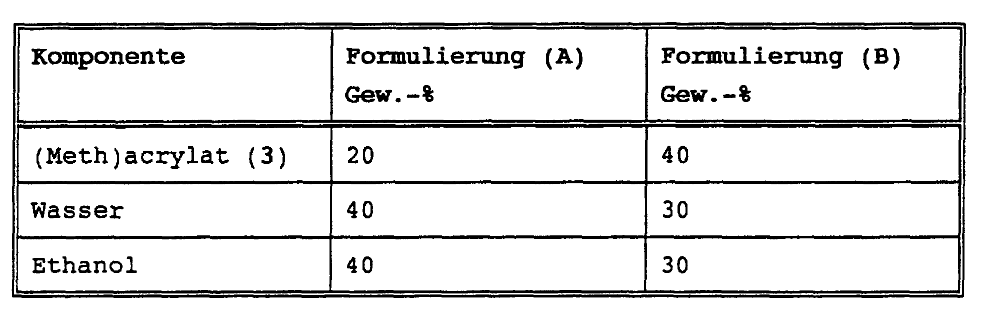

Die mit den obigen Formulierungen (A) und (B) erzielten Dentinscherhaftwerte als 3-Schichtsystem mit der Adhäsiv-Komponente und dem Bonding waren wie folgt:
- Formulierung (A):: 15,2 ± 10,5 MPa (4 von 8 Brüchen kohäsiv.
- Formulierung (B):: 21,8 ± 10,6 MPa (3 von 8 Brüchen kohäsiv)

Dazu ist anzumerken, daß bei einem kohäsiven Bruch die Bruchbildung im Substrat, d.h. im Dentin, oder im Bereich des lichtgehärteten Komposites erfolgt, während bei einem adhäsiven Bruch die Bruchzone in der Adhäsiv-Schicht liegt. Demzufolge sprechen kohäsive Brüche für eine ausgezeichnete Verbundfestigkeit des Dentinadhäsivs.

### Beispiel 11: Formulierungen mit (Meth)acrylat (3) und HEMA als funktionellen Bestandteilen

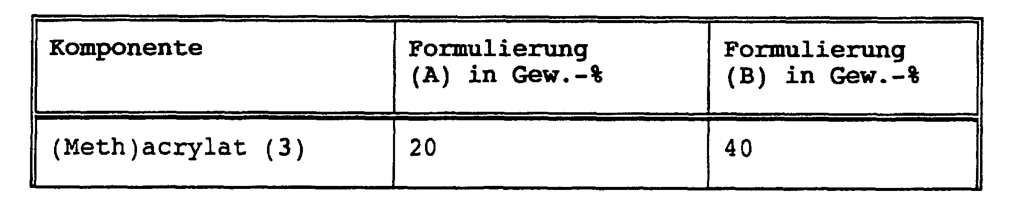

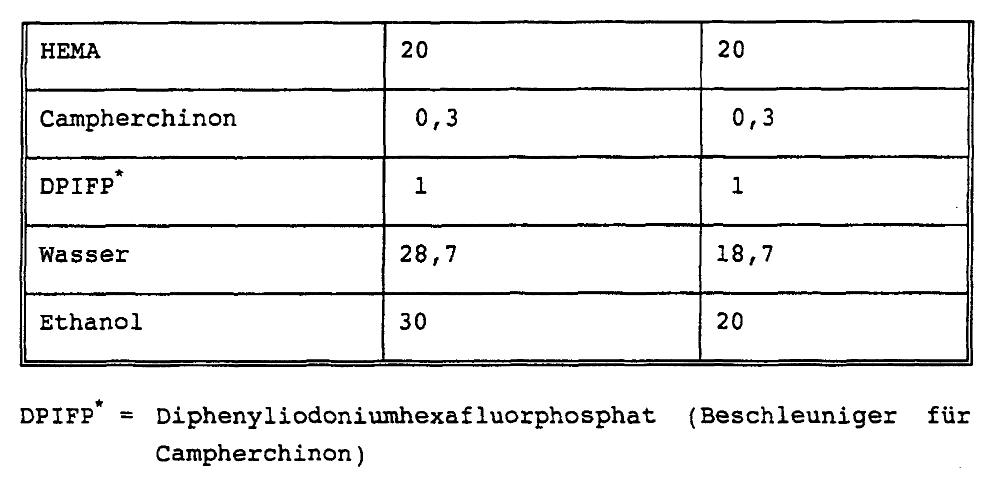

Die mit den obigen Formulierungen (A) und (B) erzielten Dentinscherhaftwerte als 2-Schichtsystem mit dem Bonding waren wie folgt:
- Formulierung (A):: 17 ± 4 MPa (2 von 6 Brüchen kohäsiv)
- Formulierung (B):: 10,1 ± 3,5 MPa (alle adhäsiv)

### Beispiel 12: Formulierungen mit (Meth)acrylat (2) als einzigem funktionellen Bestandteil

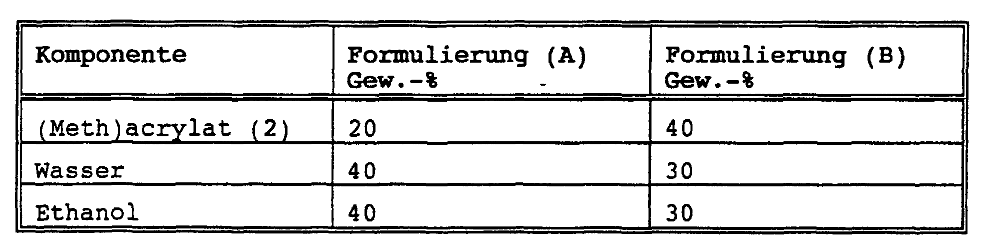

Die mit den obigen Formulierungen (A) und (B) erzielten Dentinscherhaftwerte als 3-Schichtsystem waren wie folgt:
- Formulierung (A):: 10,2 ± 8,2 MPa (2 von 7 Brüchen kohäsiv)
- Formulierung (B):: 12,3 ± 8,9 MPa (2 von 8 Brüchen kohäsiv)

Die mit Formulierung (B) erzielten Dentinscherhaftwerte als 2-Schichtsystem mit modifiziertem Bonding waren wie folgt:
- Formulierung (B):: 19,5 ± 8,2 MPa (5 von 6 Brüchen kohäsiv)

### Beispiel 13: Formulierungen mit (Meth)acrylat (2) und HEMA als funktionellen Bestandteilen

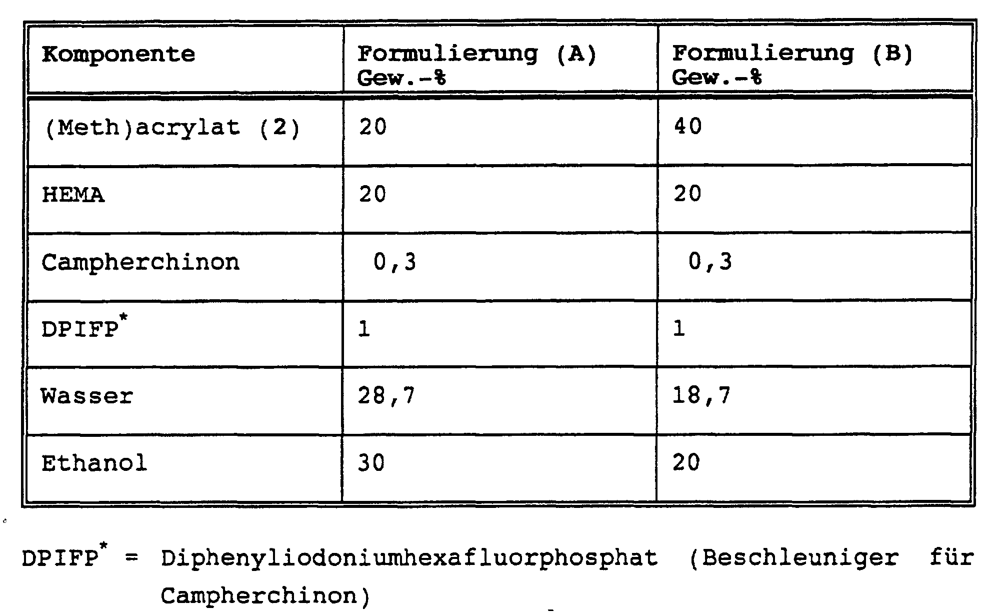

Die mit den obigen Formulierungen (A) und (B) erzielten Dentinscherhaftwerte als 2-Schichtsystem mit dem Bonding waren wie folgt:
- Formulierung (A):: 22,9 ± 8 MPa (6 von 8 Brüchen kohäsiv)
- Formulierung (B):: 16,4 ± 5 MPa (5 von 8 Brüchen kohäsiv)

### Beispiel 14: Formulierungen mit Hydroxyethylmethacrylat (HEMA) als einzigem funktionellen Bestandteil - (Vergleichsbeispiel)

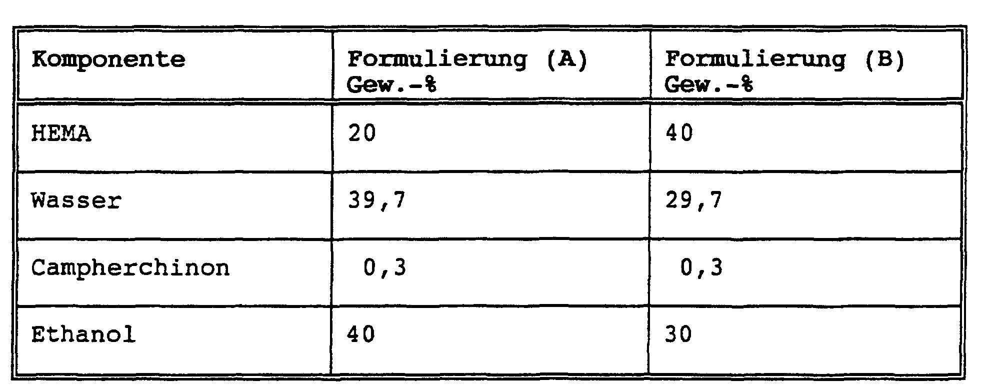

Die mit den obigen Formulierungen (A) und (B) erzielten Dentinscherhaftwerte als 3-Schichtsystem mit der Adhäsiv-Komponente und dem Bonding waren wie folgt:
- Formulierung (A):: 4,8 ± 3,2 MPa (alle Brüche adhäsiv)
- Formulierung (B):: 3,8 ± 4,1 MPa (alle Brüche adhäsiv)

Die mit den obigen Formulierungen (A) und (B) erzielten Dentinscherhaftwerte als 2-Schichtsystem mit dem Bonding waren wie folgt:
- Formulierung (A):: 2,5 ± 2,7 MPa (alle Brüche adhäsiv)
- Formulierung (B):: 2,0 ± 0,9 MPa (alle Brüche adhäsiv)

Ein Vergleich der oben angegebenen Scherhaftwerte mit den für die erfindungsgemäßen Materialien gemäß Beispiel 10 erzielten Werten zeigt die Überlegenheit der erfindungsgemäßen Materialien.

## Patentansprüche

1. Funktionalisierte bicyclische (Meth)acrylate der folgenden Formeln I oder II, dazu stereoisomere Verbindungen und Mischungen von diesen, 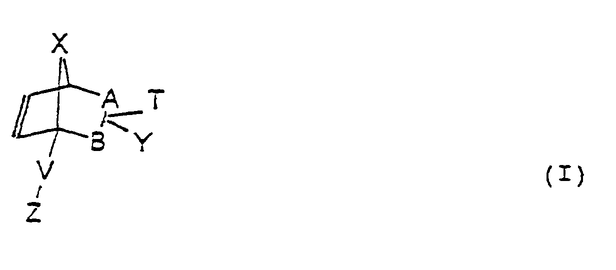 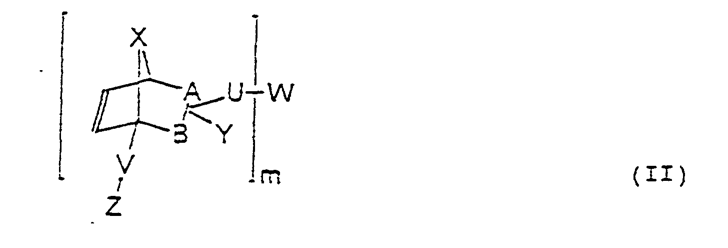 wobei A-B, T, U, V, W, X, Y, Z, R, R¹, R², R³, R⁴, n und m unabhängig voneinander die folgenden Bedeutungen haben:
A-B = C-C oder C=C;
X = CH₂, O, N-CO-OR, N-COR, N-CONR₂ oder N-SO₂R,
wobei die einzelnen Reste R unabhängig voneinander = substituiertes oder unsubstituiertes C₁- bis C₁₂-Alkyl oder C₆- bis C₁₄-Aryl;
Z = CH₂=CH-CO- oder CH₂=C(CH₃)-CO-;
V = C₁- bis C₆-Alkylenoxy, CH₂-S, CH₂-NH oder COO-(C₁-bis C₆)-Alkylenoxy;
Y = H, C₁- bis C₁₂-Alkyl, C₆- bis C₁₄-Aryl, Halogen, NO₂, NR¹₂, OR¹, CN, CO-R¹, CO-NR¹₂, CO-OR¹, SR¹, SO₂R¹ oder SO₃R¹,
wobei
die einzelnen Reste R¹ unabhängig voneinander = H, substituiertes oder unsubstituiertes C₁- bis C₁₂-Alkyl, C₆- bis C₁₄-Aryl oder -(CH₂CH₂O)ₙH mit n = 1 bis 10;
T = C₁ bis C₁₂-Alkyl, C₆- bis C₁₄-Aryl, Halogen, NO₂, NR²₂, OR², CN, CO-R², CO-NR²₂, CO-OR², SR², SO₂R² oder SO₃R²,
wobei
die einzelnen Reste R² unabhängig voneinander = H, substituiertes oder unsubstituiertes C₁- bis C₁₂-Alkyl, C₆- bis C₁₄-Aryl oder -(CH₂CH₂O)ₙH mit n = 1 bis 10;
oder Y und T gemeinsam = -CO-O-CO- oder -CO-NR³-CO-,
wobei
R³ = H, substituiertes oder unsubstituiertes C₁- bis C₁₂-Alkyl, C₆- bis C₁₄-Aryl oder -(CH₂CH₂O)ₙH mit n = 1 bis 10;
U = C₁ bis C₁₂-Alkylenoxy, CO-NR⁴-, CO-O oder O,
wobei
R⁴ = H, substituiertes oder unsubstituiertes C₁- bis C₁₂-Alkyl oder C₆- bis C₁₄-Aryl;
W = m-fach substituiertes C₁- bis C₁₂-Alkylen, C₆- bis C₁₄-Arylen, C₈- bis C₁₆-Aralkylen oder
(-CH₂CH₂OCH₂CH₂-)ₙ mit n = 1 bis 10,
wobei vorstehende Reste noch weitere Substituenten haben können;
m = 2 bis 4; und
wobei
die bei den Resten R, R¹, R², R³ und R⁴ gegebenenfalls vorhandenen Substituenten und die bei W gegebenenfalls vorhandenen weiteren Substituenten COOH, OH, Halogen, C₁- bis C₁₂-Alkoxy, -N⁺-(C₁- bis C₁₂-Alkyl)₃, -O-P=O(OH)₂ oder -P=O(OH)₂ sind und wobei bei mehreren Substituenten an den Resten, diese Substituenten unabhängig voneinander gewählt werden können.

2. Bicyclische (Meth)acrylate nach Anspruch 1, **dadurch gekennzeichnet**, daß die Variablen der Formeln I und II unabhängig voneinander die folgende Bedeutung haben:
A-B = C-C oder C=C,
X = O;
Z = CH2=CH-CO- oder CH₂=C(CH₃)-CO-;
V = CH₂-O;
Y = H, OH, COOH, CO-NH₂ oder CO-OR¹,
R¹ = H, substituiertes oder unsubstituiertes C₁- bis C₁₂-Alkyl oder C₆- bis C₁₄-Aryl;
T = OH, COOH, CO-NH₂ oder CO-OR²;
R² = H, substituiertes oder unsubstituiertes C₁- bis C₁₂-Alkyl oder C₆- bis C₁₄-Aryl;
oder Y und T gemeinsam = -CO-O-CO- oder CO-NR³-CO-;
R³ = H, substituiertes oder unsubstituiertes C₁- bis C₁₂-Alkyl oder C₆- bis C₁₄-Aryl;
U = CO-O oder CO-NR⁴;
R⁴ = H oder C₁- bis C₅-Alkyl;
W = m-fach substituiertes C₁- bis C₁₂-Alkylen, welches noch weitere Substituenten haben kann; und/oder
m = 2.

3. Bicyclische (Meth)acrylate nach Anspruch 1 oder 2, **dadurch gekennzeichnet**, daß
A-B = C-C oder C=C,
X = O und
V = CH₂O.

4. Bicyclische (Meth)acrylate nach einem der Ansprüche 1 bis 3,
**dadurch gekennzeichnet**, daß
Z = CH₂=C(CH₃)-CO-.

5. Bicyclische (Meth)acrylate nach einem der Ansprüche 1 bis 4,
**dadurch gekennzeichnet**, daß
Y und T gemeinsam = -CO-O-CO-
oder
Y = COOH und T = COOH.

6. Bicyclische (Meth)acrylate nach einem der Ansprüche 1 bis 5,
**dadurch gekennzeichnet**, daß
T = COOH und
Y = COOR¹,
wobei R¹ = mit einer oder zwei OH-Gruppen substituiertes C₁- bis C₆-Alkyl.

7. Bicyclische (Meth)acrylate nach einem der Ansprüche 1 bis 6,
**dadurch gekennzeichnet**, daß
Y = COOH,
U = CO-O und
m = 2.

8. Bicyclische (Meth)acrylate nach einem der Ansprüche 1 bis 7,
**dadurch gekennzeichnet**, daß
W = m-fach substituiertes C₁- bis C₁₂-Alkylen, das mindestens eine OH- oder mindestens eine COOH-Gruppe als weitere Substituenten tragen kann.

9. Verfahren zur Herstellung der bicylischen (Meth)acrylate gemäß einem der Ansprüche 1 bis 8, dadurch gekennzeichnet, daß man
(a) eine substituierte Dien(meth)acryl-Verbindung der allgemeinen Formel (III) mit einem substituierten Dienophil der allgemeinen Formel (IV) unter Bildung des bicyclischen (Meth)acrylats der Formel (I) im Rahmen einer Diels-Alder-Reaktion entsprechend nachstehender Reaktionsgleichung umsetzt, 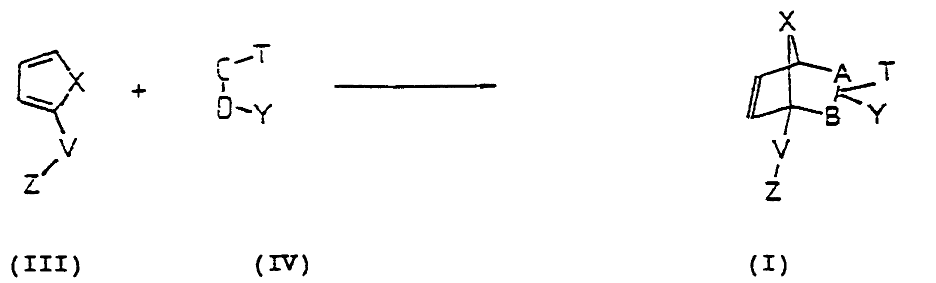 wobei
C-D = C=C oder C≡C und
die übrigen Variablen wie in Anspruch 1 definiert sind,
oder
(b) eine bicyclische Verbindung der Formel (V) mit einer Polyhydroxyverbindung der Formel (VI) entsprechend nachstehender Reaktionsgleichung zum (Meth)acrylat der Formel (II) kondensiert, 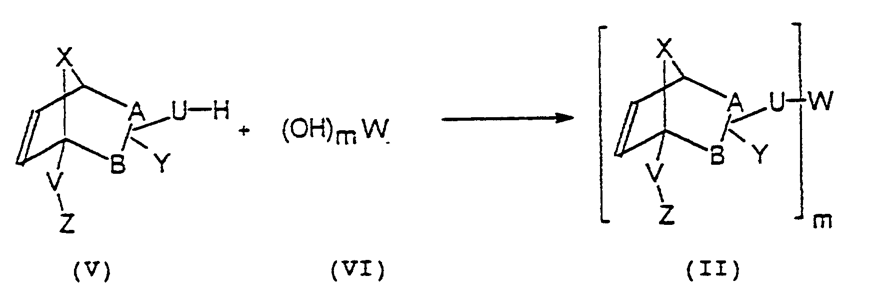 wobei die Variablen wie in Anspruch 1 definiert sind.

10. Verwendung der bicyclischen (Meth)acrylate gemäß einem der Ansprüche 1 bis 8 zur Herstellung von Dentalmaterial oder einem Bestandteil von Dentalmaterial.

11. Verwendung nach Anspruch 10, wobei das Dentalmaterial ein Dentinadhäsiv ist.

12. Dentalmaterial mit Gehalt an polymerisiertem und/oder nichtpolymerisiertem bicyclischen (Meth)acrylat gemäß einem der Ansprüche 1 bis 8.

13. Dentalmaterial nach Anspruch 12, **dadurch gekennzeichnet**, daß es ein Dentinadhäsiv ist.

14. Polymere oder Copolymere, **dadurch gekennzeichnet**, daß sie durch Polymerisation oder Copolymerisation der bicyclischen (Meth)acrylate gemäß einem der Ansprüche 1 bis 8 erhältlich sind.

## Claims

1. Functionalized bicyclic (meth)acrylates having the following formulae I or II, and also stereoisomeric compounds and mixtures thereof, 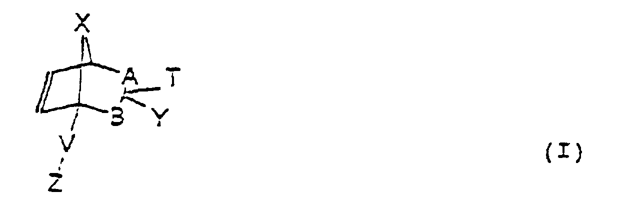 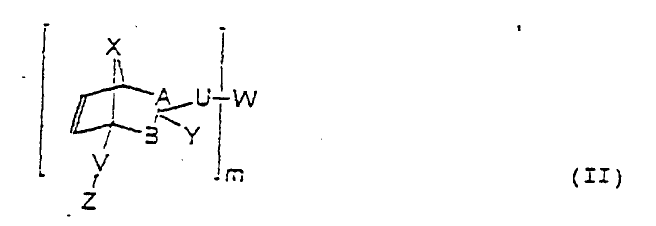 wherein A-B, T, U, V, W, X, Y, Z, R, R¹, R², R³, R⁴, n and m, independently of one another, have the following meanings:
A-B = C-C or C=C;
X = CH₂, O, N-CO-OR, N-COR, N-CONR₂ or N-SO₂R,
where
the individual radicals R independently of one another = substituted or unsubstituted C₁- to C₁₂-alkyl or C₆- to C₁₄-aryl;
Z = CH₂=CH=CO- or CH₂=C(CH₃)-CO-;
V = C₁- to C₆-alkyleneoxy, CH₂-S, CH₂-NH or COO-(C₁- to C₆)-alkyleneoxy;
Y = H, C₁- to C₁₂-alkyl, C₆- to C₁₄-aryl, halogen, NO_{2,} NR¹₂, OR¹, CN, CO-R¹, CO-NR¹₂, CO-OR¹, SR¹, SO₂R¹ or SO₃R¹,
where
the individual radicals R¹ independently of one another = H, substituted or unsubstituted C₁- to C₁₂-alkyl, C₆- to C₁₄-aryl or -(CH₂CH₂O)ₙH with n = 1 to 10;
T = C₁- to C₁₂-alkyl, C₆- to C₁₄-aryl, halogen, NO₂, NR²₂, OR², CN, CO-R², CO-NR²₂, CO-OR², SR², SO₂R² or SO₃R²,
where
the individual radicals R² independently of one another = H, substituted or unsubstituted C₁- to C₁₂-alkyl, C6- to C₁₄-aryl or - (CH₂CH₂O)ₙH with n = 1 to 10;
or Y and T together = -CO-O-CO- or -CO-NR³-CO-,
where
R³ = H, substituted or unsubstituted C₁-to C₁₂-alkyl, C₆- to C₁₄-aryl or -(CH₂CH₂O)ₙH with n = 1 to 10;
U = C₁- to C₁₂-alkyleneoxy, CO-NR⁴-, CO-O or O,
where
R⁴ = H, substituted or unsubstituted C₁-to C₁₂-alkyl or C₆- to C₁₄-aryl;
W = m-fold substituted C₁- to C₁₂-alkylene, C₆-to C₁₄-arylene, C₈- to C₁₆-aralkylene or (-CH₂CH₂OCH₂CH₂-)ₙ with n = 1 to 10,
where the abovementioned radicals may have further substituents;
m = 2 to 4 and
wherein
the substituents optionally present for the radicals R, R1, R2, R3 and R4 and the further substituents optionally present for W are COOH, OH halogen, C1- to C₁₂-alkoxy, -N⁺-(C₁- to C₁₂-alkyl)₃, - O-P=O(OH)₂ or - P=O(OH)₂₎ and where there are a plurality of substituents on the radicals these substituents can be selected independently of one another.

2. Bicyclic (meth)acrylates according to Claim 1, characterized in that the variables of the formulae I and II, independently of one another, have the following meaning:
A-B = C-C or C=C;
X = O;
Z = CH₂=CH-CO- or CH₂=C(CH₃)-CO-;
V = CH₂-O;
Y = H, OH, COOH, CO-NH₂ or CO-OR¹;
R¹ = H, substituted or unsubstituted C₁- to C₁₂-alkyl or C₆- to C₁₄-aryl;
T = OH, COOH, CO-NH₂ or CO-OR²;
R² = H, substituted or unsubstituted C₁- to C₁₂-alkyl or C₆- to C₁₄-aryl;
or Y and T together = -CO-O-CO- or CO-NR³-CO-;
R³ = H, substituted or unsubstituted C₁- to C₁₂-alkyl or C₆- to C₁₄-aryl;
U = CO-O or CO-NR⁴;
R⁴ = H or C₁- to C₅-alkyl;
W = m-fold substituted C₁- to C₁₂-alkylene, which may have further substituents; and/or
m = 2.

3. Bicyclic (meth)acrylates according to claim 1 or 2, characterized in that
A-B = C-C or C=C,
X = O and
V = CH₂O.

4. Bicyclic (meth)acrylates according to any one of Claims 1 to 3, characterized in that
Z = CH₂=C(CH₃)-CO-.

5. Bicyclic (meth)acrylates according to any one of Claims 1 to 4, characterized in that
Y and T together = -CO-O-CO-
or
Y = COOH and T = COOH.

6. Bicyclic (meth)acrylates according to any one of Claims 1 to 5, characterized in that
T = COOH and
Y = COOR¹
where R¹ = C₁- to C₆-alkyl substituted with one or two OH groups.

7. Bicyclic (meth)acrylates according to any one of Claims 1 to 6, characterized in that
Y = COOH,
U = CO-O and
m = 2.

8. Bicyclic (meth)acrylates according to any one of Claims 1 to 7, characterized in that
W = m-fold substituted C₁- to C₁₂-alkylene, which may have at least one OH or at least one COOH group as further substituents.

9. Process for the preparation of the bicyclic (meth)acrylates according to any one of Claims 1 to 8, characterized in that
(a) a substituted diene(meth)acrylic compound having the general formula (III) is reacted with a substituted dienophile having the general formula (IV) with formation of the bicyclic (meth)acrylate having the formula (I) by way of a Diels-Alder reaction in accordance with the reaction equation below: 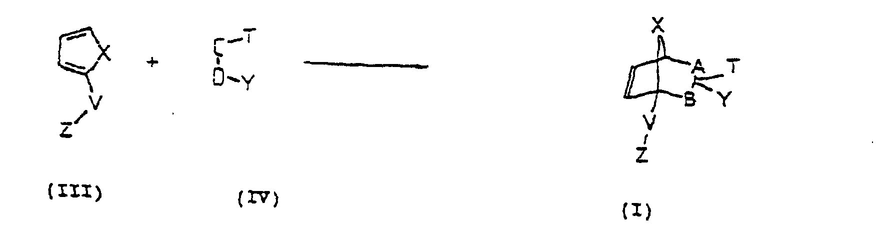 where
C-D = C=C or C≡C and
the other variables are as defined in Claim 1,
or
(b) a bicyclic compound having the formula (V) is condensed with a polyhydroxyl compound having the formula (VI) in accordance with the reaction equation below to form the (meth)acrylate having the formula (II), 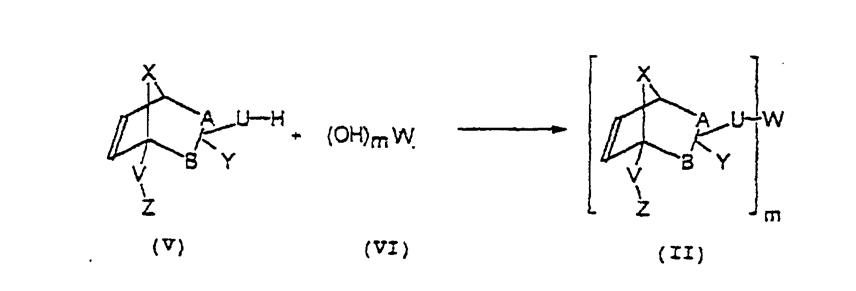 where the variables are as defined in Claim 1.

10. Use of the bicyclic (meth)acrylates according to any one of Claims 1 to 8 for the production of dental material or a constituent of dental material.

11. Use according to Claim 10, wherefor the dental material is a dentine adhesive.

12. Dental material containing polymerized and/or non-polymerized bicyclic (meth)acrylate according to any one of claims 1 to 8.

13. Dental material according to Claim 12, characterized in that it is a dentine adhesive.

14. Polymers or copolymers characterized in that they are obtainable by polymerization or copolymerization of the bicyclic (meth)acrylates according to any one of Claims 1 to 8.

## Revendications

1. (Méth)acrylates bicycliques fonctionnalisés de formule I ou II ci-après, et composés stéréoisomères et mélanges de ceux-ci 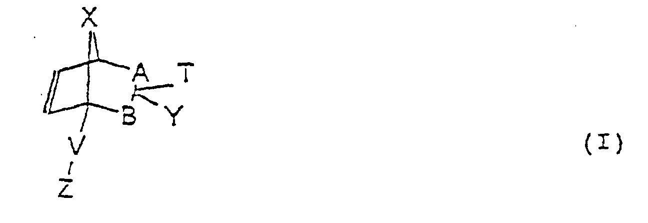 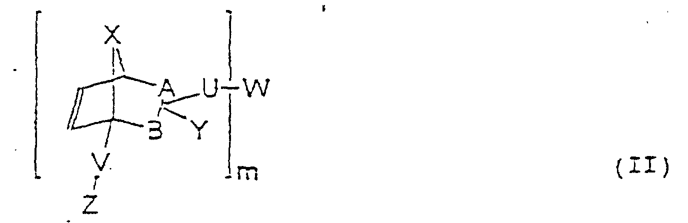 formules dans lesquelles A-B, T, U, V, W, X, Y, Z, R, R¹, R², R³, R⁴, n et m, indépendamment les uns des autres, ont les significations suivantes :
A-B représente C-C ou C=C ;
X représente CH₂, O, N-CO-OR, N-COR, N-CONR₂ ou N-SO₂R, les radicaux R individuels représentant, indépendamment les uns des autres, un groupe alkyle en C₁-C₁₂ ou aryle en C₆-C₁₄, substitués ou non substitués ;
Z représente CH₂=CH-CO- ou CH₂=C(CH₃)-CO-;
V représente un groupe alkylènoxy en C₁-C₆, CH₂-S, CH₂-NH ou COO- alkylènoxy(C₁-C₆) ;
Y représente H ou un groupe alkyle en C₁-C₁₂, aryle en C₆-C₁₄, un atome d'halogène, un groupe NO₂, NR¹₂, OR¹, CN, CO-R¹, CO-NR¹₂, CO-OR¹, SR¹, SO₂R¹ ou SO₃R¹,
les radicaux R¹ individuels représentant, indépendamment les uns des autres, H ou un groupe alkyle en C₁-C₁₂, aryle en C₆-C₁₄, substitués ou non substitués, ou -(CH₂CH₂O)ₙH dans lequel n = 1 à 10;
T représente un groupe alkyle en C₁-C₁₂, aryle en C₆-C₁₄, un atome d'halogène, un groupe NO₂, NR²₂, OR², CN, CO-R², CO-NR²₂, CO-OR², SR², SO₂R² ou SO₃R²,
les radicaux R² individuels représentant, indépendamment les uns des autres, H ou un groupe alkyle en C₁-C₁₂, aryle en C₆-C₁₄, substitués ou non substitués, ou -(CH₂CH₂O)ₙH, n allant de 1 à 10 ;
ou Y et T forment ensemble un groupe -CO-O-CO- ou -CO-NR³-CO-,
R³ représentant H ou un groupe alkyle en C₁-C₁₂, aryle en C₆-C₁₄, substitués ou non substitués, ou -(CH₂CH₂O)ₙH, dans lequel n = 1 à 10;
U représente un groupe alkylènoxy en C₁-C₁₂, CO-NR⁴-, CO-O ou O, R⁴ représentant H ou un groupe alkyle en C₁-C₁₂ ou aryle en C₆-C₁₄, substitués ou non substitués,
W représente un groupe alkylène en C₁-C₁₂, arylène en C₆-C₁₄, aralkylène en C₈-C₁₆, m-fois substitués, ou (-CH₂CH₂OCH₂CH₂-)ₙ dans lequel n va de 1 à 10,
les radicaux précédents pouvant porter encore d'autres substituants ;
m = 2 à 4 ; et
les substituants éventuellement présents sur les radicaux R, R¹, R², R³ et R⁴ et les autres substituants éventuellement présents sur W étant des atomes d'halogène ou des groupes COOH, OH, alcoxy en C₁-C₁₂, -N⁺-[alkyle(C₁-C₁₂)]₃, -O-P=O(OH)₂ ou -P=O(OH)₂ et, dans le cas de plusieurs substituants sur les radicaux, ces substituants pouvant être choisis indépendamment les uns de autres.

2. (Méth)acrylates bicycliques selon la revendication 1, caractérisés en ce que les variables des formules I et II ont, indépendamment les unes des autres, les significations suivantes :
A-B = C-C ou C=C ;
X = O ;
Z = CH₂=CH-CO- ou CH₂=C(CH₃)-CO- ;
V = CH₂-O;
Y = H, OH, COOH, CO-NH₂ ou CO-OR¹ ;
R¹ = H ou un groupe alkyle en C₁-C₁₂ ou aryle en C₆-C₁₄, substitués ou non substitués ;
T = OH, COOH, CO-NH₂ ou CO-OR²;
R² = H ou un groupe alkyle en C₁-C₁₂ ou aryle en C₆-C₁₄, substitués ou non substitués ;
ou Y et T forment ensemble -CO-O-CO- ou CO-NR³-CO- ;
R³ = H ou un groupe alkyle en C₁-C₁₂ ou aryle en C₆-C₁₄, substitués ou non substitués ;
U = CO-O ou CO-NR⁴ ;
R⁴ = H ou un groupe alkyle en C₁-C₅;
W = un groupe alkylène en C₁-C₁₂ m-fois substitué, qui peut porter encore d'autres substituants ; et/ou
m = 2.

3. (Méth)acrylates bicycliques selon la revendication 1 ou 2 caractérisés en ce que
A-B = C-C ou C=C,
X = O et
V = CH₂O.

4. (Méth)acrylates bicycliques selon l'une des revendications 1 à 3, caractérisés en ce que
Z = CH₂=C(CH₃)-CO-.

5. (Méth)acrylates bicycliques selon l'une des revendications 1 à 4, caractérisés en ce que
Y et T forment ensemble -CO-O-CO-
ou
Y = COOH et T = COOH.

6. (Méth)acrylates bicycliques selon l'une des revendications 1 à 5, caractérisés en ce que
T = COOH et
Y = COOR¹,
R¹ représentant un groupe alkyle en C₁-C₆ substitué par un ou deux groupes OH.

7. (Méth)acrylates bicycliques selon l'une des revendications 1 à 6, caractérisés en ce que
Y = COOH,
U = CO-O et
m = 2.

8. (Méth)acrylates bicycliques selon l'une des revendications 1 à 7, caractérisés en ce que
W représente un groupe alkylène en C₁-C₁₂ m-fois substitué, qui peut porter comme autres substituants au moins un groupe OH ou au moins un groupe COOH.

9. Procédé pour la préparation des (méth)acrylates bicycliques selon l'une des revendications 1 à 8, caractérisé en ce que
(a) on fait réagir un composé diène (méth)acrylique substitué de formule générale (III) avec un composé diénophile substitué de formule générale (IV), avec formation du (méth)acrylate bicyclique de formule (I), dans le cadre d'une réaction de Diels-Alder, selon l'équation de réaction ci-après, 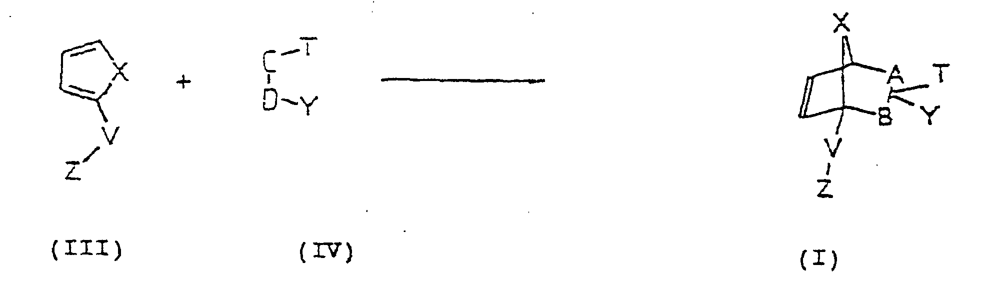 dans laquelle C-D = C=C ou C≡C et
les autres variables sont telles que définies dans la revendication 1, ou
(b) on condense un composé bicyclique de formule (V) avec un composé polyhydroxylé de formule (VI), selon l'équation de réaction ci-après, pour aboutir au (méth)acrylate de formule (II) 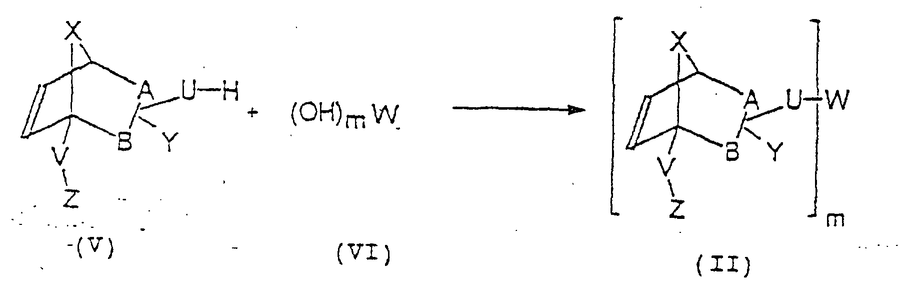 les variables étant telles que définies dans la revendication 1.

10. Utilisation des (méth)acrylates bicycliques selon l'une des revendications 1 à 8, pour la fabrication de matériau dentaire ou d'un composant de matériau dentaire.

11. Utilisation selon la revendication 10, dans laquelle le matériau dentaire est un adhésif pour dentine.

12. Matériau dentaire ayant une teneur en (méth)acrylate bicyclique polymérisé et/ou non polymérisé selon l'une des revendications 1 à 8.

13. Matériau dentaire selon la revendication 12, caractérisé en ce qu'il est un adhésif pour dentine.

14. Polymères ou copolymères, caractérisés en ce qu'ils peuvent être obtenus par polymérisation ou copolymérisation des (méth)acrylates bicycliques selon l'une des revendications 1 à 8.
